# EUROPÄISCHE PATENTANMELDUNG

(11) **EP 3 822 348 A1**
(43) Veröffentlichungstag der Anmeldung: **19.05.2021**
(21) Anmeldenummer: 19208603.1
(22) Anmeldetag: 12.11.2019
(51) Int. Cl.: C12N 9/00

(54) **ADAPTERSYSTEM ARTIFIZIELLER NICHTRIBOSOMALER PEPTIDSYNTHETASEN UND POLYKETIDSYNTHASEN**

(71) Anmelder: Johann Wolfgang Goethe-Universität Frankfurt am Main, 60323 Frankfurt am Main (DE)
(72) Erfinder: BODE, Helge, 61440 Oberursel (DE); BOZHÜYÜK, Kenan, 60594 Frankfurt am Main (DE); WATZEL, Jonas, 55131 Mainz (DE)
(74) Vertreter: Kuttenkeuler, David

(57) **Zusammenfassung**

Die Erfindung betrifft ein System zur Expression von nichtribosomale Peptidsynthetasen (NRPSs), Polyketidsynthasen (PKS) oder NRPS/PKS-Hybridsynth(et)asen. NRPS, PKS oder deren Hybride sind große Multidomänenproteine oder Multidomänenkomplexe, deren Expression zur Herstellung von Peptiden oft Schwierigkeiten bereitet. Entsprechend betrifft die Erfindung ein System zur Expression von Teilstücken der Enzyme, die post-translational über gezielt eingeführte Protein-Protein Wechselwirkungen zu funktionellen Multienzymkomplexen zusammengebaut werden können. Die Erfindung offenbart Proteinfragmente eines solchen Bausatzes, sowie deren kodierende Nukleinäuren. Ebenfalls offenbart ist ein Vektorensystem der Proteinfragmente der Erfindung und dessen Verwendung zur Herstellung funktioneller NRPS/PKS Enzymkomplexe.

## Beschreibung

### GEBIET DER ERFINDUNG

Die Erfindung betrifft ein System zur Expression von nichtribosomale Peptidsynthetasen (NRPSs), Polyketidsynthasen (PKS) oder NRPS/PKS-Hybridsynth(et)asen. NRPS, PKS oder deren Hybride sind große Multidomänenproteine oder Multidomänenkomplexe, deren Expression zur Herstellung von Peptiden oft Schwierigkeiten bereitet. Entsprechend betrifft die Erfindung ein System zur Expression von Teilstücken der Enzyme, die post-translational über gezielt eingeführte Protein-Protein Wechselwirkungen zu funktionellen Multienzymkomplexen zusammengebaut werden können. Die Erfindung offenbart Proteinfragmente eines solchen Bausatzes, sowie deren kodierende Nukleinäuren. Ebenfalls offenbart ist ein Vektorensystem der Proteinfragmente der Erfindung und dessen Verwendung zur Herstellung funktioneller NRPS/PKS Enzymkomplexe.

### BESCHREIBUNG

Nichtribosomale Peptide (NRP) sind durch nichtribosomale Peptidsynthetasen (NRPSs) produzierte Peptide mit einer großen strukturellen Diversität, die sich insbesondere durch zyklische, verzweigte oder andere komplexe Primärstrukturen kennzeichnen (Caradec et al., 2014; Caboche et al., 2010). Viele dieser Moleküle zeigen, aufgrund ihrer strukturellen Komplexität, therapeutische Eigenschaften zu denen antibiotische, immunsuppressive oder antikanzerogenen Wirkweisen zählen (Finking und Marahiel, 2004; Felnagle et al., 2008). Sie sind dadurch nicht nur Quelle neuer Medikamente, sondern dienen auch als Grundgerüste für pharmazeutische Reagenzien (Cane et al., 1998). Meist werden zu diesem Zweck die Naturstoffe durch Semisynthese, einem Prozess der Biosynthese und organische Synthese verbindet, chemisch modifiziert (Kirschning und Hahn, 2012). Alternativ können neuartige NRPs auch durch die Reprogrammierung der für die Synthese verantwortlichen NRPSs produziert werden. Hierzu wird oft der modulare Aufbau der Synthetasen ausgenutzt und diese auf genetischer Ebene manipuliert. In der Literatur sind einige Beispiele bekannt bei denen die Reprogrammierung von NRPSs erfolgreich war (Schneider et al, 1998; Chiocchini et al., 2006). Nichtsdestotrotz ist die Produktivität dieser Synthetasen meist stark eingeschränkt (Suo, 2005).

Die strukturelle und funktionelle Diversität von NRPs kommt durch den Einbau von D-Aminosäuren (AS), heterozyklischen Elementen oder N-methylierten Seitenketten sowie durch das Anfügen von Fetten, Zuckern und Halogenen zustande (Hur et al., 2012). Beispiele für NRPs mit solchen strukturellen Besonderheiten sind in etwa Bacitracin und Vibriobactin, die heterozyklische Ringe tragen oder Cyclosporin A und Tyrocidin A, die durch die Inkorporation von D-AS gekennzeichnet sind. Daptomycin dagegen ist ein acetyliertes Peptid, das aufgrund seiner Fettsäure eine starke antibakterielle Wirkung aufweist; wohingegen Balhimycin und Syringomycin Beispiele für halogenierte NRPs mit antibakterillen und antifungalen Eigenschaften sind.

SYNZIPs sind heterospezifische synthetische coiled-coils, die eine kontrollierte Protein-Interaktion ermöglichen und Anwendung in der synthetischen Biologie finden. Coiled-coils setzen sich im Allgemeinen aus zwei, drei oder vier amphipathischen 20-50 AS langen α-Helices zusammen, die einen ineinander gewunden linksgängigen Supercoil bilden. Sie sind Strukturmotiv vieler Proteine und treten bspw. auch in den Leucin-Zipper-Regionen humaner bZIP Transkriptionsfaktor auf. Coiled-coils kennzeichnen sich durch ein Heptadenmuster (abcdefg)ₙ, die an den Positionen a und d hydrophobe AS tragen, während auf den Positionen e und g meistens elektrostatische AS vorkommen. In einer α-helikalen Sekundärstruktur interagieren diese hydrophoben AS miteinander und formen ein enges hydrophobes Interface (Lumb et al., 1994).

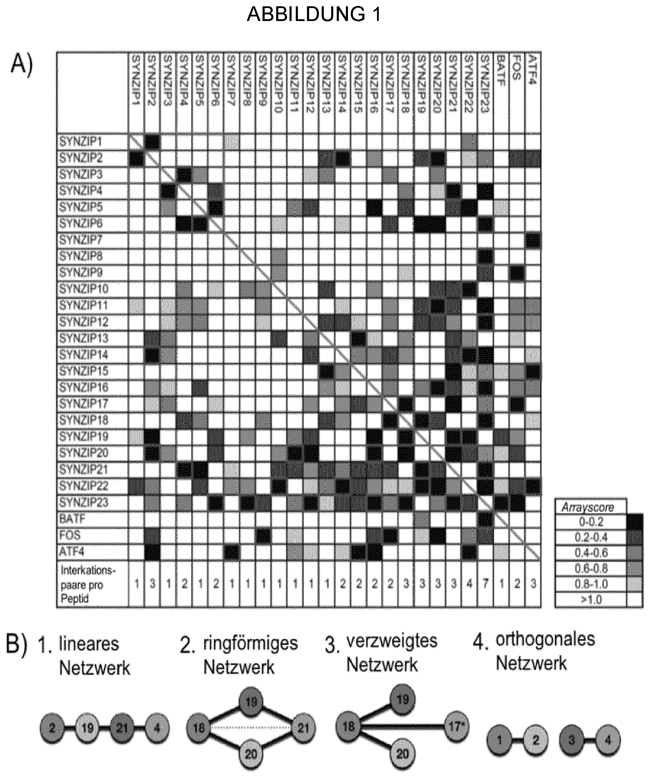

Ursprünglich wurden SYNZIPs für die heterospezifisch Interaktion mit Leucin-Zipper-Regionen humaner bZIP Trankriptionsfaktoren (TF) entwickelt. Hierfür wurden computerbasiert 48 künstliche Peptide konstruiert, die anschließend hinsichtlich ihrer Interaktion mit diesen untersucht wurden (Grigoryan et al., 2009). In einer weiteren Studie dagegen wurde die Interaktion der Peptide auch untereinander geprüft. Dafür führte Reinke et al. ein Protein-Mikroarray-Assay durch, bei welchem alle 48 künstlichen sowie 7 weitere coiled-coils humaner bZIPs gegeneinander getestet wurden (Abb. 1). Aus den Ergebnissen des Assays wurden 27 Paare, 23 synthetische (namentlich SYNZIP 1-23) und drei humane bZIP-Strukturen ausgewählt, die eine starke heterospezifische und gleichzeitig geringe homospezfische Interaktion zeigten. Wie in Abb. 1A zu entnehmen ist, sind die Peptide an mindestens einer bis maximal sieben Interaktionen beteiligt und können in einigen Fällen unterschiedliche Netzwerke bilden (Abb. 1 B). Beispiele hierfür sind lineare, ringförmige, verzweigte und orthogonale Netzwerke (Thompson et al., 2012). Des Weiteren wurde aus der Asn-Asn Paarung auf den a-a' Positionen darauf geschlossen, dass es sich bei den meisten Paaren um parallele Heterodimere handeln muss (Reinke et al., 2010).

Die internationale Veröffentlichung WO 2019/138117 beschreibt ein System zum Zusammenbau und zur Modifikation von NRPS. Das System verwendet neuartige, genau definierte Bausteine (Einheiten), die Kondensationssubdomänen umfassen. Diese Strategie ermöglicht die effiziente Kombination von Baugruppen, die als eXchange Units (XU2.0) bezeichnet werden, unabhängig von ihrer natürlich vorkommenden Spezifität für die nachfolgende NRPS-Adenylierungsdomäne. Das System der WO 2019/138117 ermöglicht den einfachen Zusammenbau von NRPS mit einer Aktivität für die Synthese eines Peptids mit beliebiger Aminosäuresequenz, ohne Einschränkungen aufgrund natürlich vorkommender NRPS-Einheiten. Das System ermöglicht auch den Austausch natürlicher NRPS-Bausteine mit dem erfindungsgemäßen XU2.0, wodurch modifizierte Peptide erzeugt werden. Zwar ermöglicht das System eine einfache Kombination von XU2.0 Einheiten, verlangt aber dennoch die Expression des assemblierten NRPS Proteins in einem Open Reading Frame (ORF). Dies führt insbesondere bei längeren NRPS zu Problemen.

Daher ist es die Aufgabe der vorliegenden Erfindung NRPS Module, oder Teilmodule (oder Domänen) wie beispielsweise XU2.0 Einheiten aus WO 2019/138117, effizient und flexibel rekombinant, herzustellen.

### KURZE BESCHREIBUNG DER ERFINDUNG

Im Allgemeinen und mittels einer kurzen Beschreibung können die Hauptaspekte der vorliegenden Erfindung wie folgt beschrieben werden:

In einem **ersten Aspekt** betrifft die Erfindung ein Protein oder ein Proteinfragment, umfassend mindestens eine erste Domäne oder Teildomäne einer Nicht-Ribosomalen-Peptidsynthetase (NRPS), einer Polyketidsynthase (PKS) oder einer NRPS/PKS-Hybridsynth(et)ase (erste PKS-NRPS-Domäne), wobei das Protein oder das Proteinfragment einen N-Terminus oder einen C-Terminus umfassend eine erste Bindedomäne aufweist und wobei diese erste Bindedomäne vorzugsweise den N-Terminus, respektive C-Terminus, des Proteins oder des Proteinfragments darstellt, und wobei die erste Bindedomäne durch die Eigenschaft gekennzeichnet ist eine spezifische Protein-Protein Bindung mit mindestens einer korrespondierenden zweiten Bindedomäne eingehen zu können.

In einem **zweiten Aspekt** betrifft die Erfindung ein isoliertes Nukleinsäurekonstrukt, umfassend einen ersten kodierenden Bereich, der eine Nukleinsäuresequenz aufweist, die für ein Protein oder Proteinfragment des ersten Aspekts kodiert.

In einem **dritten Aspekt** betrifft die Erfindung ein Vektorsystem zur Herstellung einer funktionellen NRPS oder PKS, wobei das Vektorsytem mindestens ein Nukleinsäurekonstrukt gemäß des zweiten Aspekts umfasst, und wobei das mindestens ein Nukleinsäurekonstrukt geeignet ist mindestens zwei Proteine oder Proteinfragmente gemäß des ersten Aspekts zu exprimieren, und wobei die mindestens zwei Proteine oder Proteinfragmente unterschiedlich sind und zusammen eine funktionelle NRPS, PKS oder NRPS/PKS-Hybrid bilden.

In einem **vierten Aspekt** betrifft die Erfindung ein Verfahren zur Herstellung einer funktionellen (vollständigen) NRPS oder PKS, umfassend das in-Kontakt bringen von mindestens einem ersten Protein oder Proteinfragment gemäß des ersten Aspekts mit einem zweiten Protein oder Proteinfragment gemäß des ersten Aspekts, wobei das erste Protein oder Proteinfragment eine terminale erste Bindedomäne aufweist, und wobei das zweite Protein oder Proteinfragment anstatt der terminalen ersten Bindedomäne die terminale zweite Bindedomäne aufweist.

### DETAILLIERTE BESCHREIBUNG DER ERFINDUNG

Im Folgenden werden die Elemente der Erfindung beschrieben. Diese Elemente werden mit spezifischen Ausführungsformen beschrieben. Es versteht sich jedoch, dass die Elemente der Erfindung auf jede Weise und in jeder Zahl miteinander kombiniert werden können, um zusätzliche Ausführungsformen zu erhalten. Die verschiedenartig beschriebenen Beispiele und bevorzugten Ausführungsformen sollten nicht so ausgelegt werden, dass sie die vorliegende Erfindung nur auf die explizit beschriebenen Ausführungsformen oder Beispiele beschränken. Die vorliegende Offenbarung sollte so verstanden werden, dass sie Ausführungsformen beschreibt und umfasst, die zwei oder mehr der explizit beschriebenen Ausführungsformen oder Elemente miteinander kombinieren oder die eine oder mehrere der explizit beschriebenen Ausführungsformen mit einer beliebigen Anzahl der offenbarten und / oder bevorzugten Elemente kombinieren. Darüber hinaus sollten alle Permutationen und Kombinationen aller in dieser Anmeldung beschriebenen Elemente als durch die Beschreibung der vorliegenden Anmeldung offenbart angesehen werden, sofern der Kontext oder der technische Zusammenhang nichts anderes angibt oder erlaubt.

Der Ausdruck "partielle Domäne" oder "partielle C- oder C / E-Domäne" oder auch "Teildomäne", oder ähnliche Begriffe, bezieht sich auf eine Nukleinsäuresequenz, die für eine NRPS-PKS-Domäne kodiert, oder eine Proteinsequenz davon, die unvollständig ist (nicht in voller Länge). Dabei ist der Begriff so zu verstehen, dass die Teildomäne im Vergleich zu der Volllängendomäne einen zusammenhängenden Anteil von mindestens 20%, vorzugsweise 30% oder 40% oder mehr, einer aufeinander folgenden Sequenz der Volllängendomäne aufweist. Eine Teildomäne weißt daher einen sehr hohen Grad der Sequenzidentität (90% und mehr) zu einem zusammenhängenden Teilstück (mindestens 20%, vorzugsweise 30% oder 40% oder mehr) der Sequenz der Volllängendomäne auf. Beispielsweise beschreibt der Ausdruck eine C- oder C / E-Domänensequenz, die nicht sowohl Donor- als auch Akzeptorstellen einer NRPS-C- oder C / E-Domäne umfasst. Teildomänen von NRPS weisen beispielsweise eine Sequenzlänge von 100 oder mehr, 150 oder mehr, oder ungefähr 200 Aminosäuren auf.

Mit "Zusammenstellung" ist eine Reihe von Domänen gemeint. Eine Vielzahl von NRPS/PKS-Baugruppen umfasst eine vollständige NRPS-PKS. Ein oder mehrere Polypeptide können ein Modul umfassen. Modulkombinationen katalysieren dann in Kombination längere Peptide. In einem Beispiel kann ein Modul eine C-Domäne (Kondensationsdomäne), eine A-Domäne (Adenylierungsdomäne) und eine Peptidylträger-Proteindomäne umfassen.

Weitere strukturelle Informationen zu A-Domänen, C-Domänen, Didomänen, Domäne-Domäne-Schnittstellen und vollständigen Modulen finden Sie bei Conti et al. (1997), Sundlov et al. (2013), Samel et al. (2007), Tanovic et al. (2008), Strieker und Marahiel (2010), Mitchell et al. (2012) und Tan et al. (2015).

Mit "Initiierungsmodul" ist ein N-terminales Modul gemeint, das ein erstes Monomer an ein anderes Modul übergeben kann (z. B. ein Verlängerungs- oder Abschlussmodul). In einigen Fällen ist das weitere Modul nicht das zweite, sondern eines der C-terminal folgenden Module (beispielsweise bei der Nocardicin-NRPS). Im Fall einer NRPS umfasst ein Initiierungsmodul beispielsweise eine A (Adenylierung) -Domäne und ein PCP (Peptidyl Carrier Protein) oder eine T (Thiolation) -Domäne. Das Initiierungsmodul kann auch eine Starter-C-Domäne und / oder eine E-Domäne (Epimerisierungsdomäne) enthalten. Bei einer PKS besteht ein mögliches Initiationsmodul aus einer AT-Domäne (Acetyltransferase) und einer ACP-Domäne (Acyl Carrier Protein). Initiierungsmodule befinden sich vorzugsweise am Aminoterminus eines Polypeptids des ersten Moduls einer "Montagereihe". Jede Montagereihe enthält vorzugsweise ein Initiierungsmodul.

Der Begriff "Verlängerungsmodul" oder "Elongationsmodul" bezeichnet ein Modul, das ein Donor-Monomer einem Akzeptor-Monomer oder einem Akzeptor-Polymer hinzufügt, und somit die Peptidkette verlängert. Ein Elongationsmodul kann eine C- (Kondensation), Cy-(Heterozyklisierung), E-, C / E-, MT- (Methyltransferase), A-MT- (kombinierte Adenylierungs- und Methylierungsdomäne), Ox- (Oxidase) oder Re- (Reduktase) Domäne; eine A-Domäne; oder eine T-Domäne umfassen. Eine Elongationsdomäne kann ferner zusätzliche E-, Re-, DH-(Dehydratisierung), MT-, NMet- (N-Methylierung), AMT- (Aminotransferase) oder Cy-Domänen umfassen. Zusätzlich könnte ein Elongationsmodul PKS-Ursprungs sein, und die jeweiligen Domänen (Ketosynthase (KS), Acyltransferase (AT), Ketoreduktase (KR), Dehydratase (DH), Enoylreduktase (ER, Thiolierung (T)) umfassen.

Als "Terminierungsmodul" wird ein Modul bezeichnet, das das Molekül (z. B. ein NRP, eine PK oder Kombinationen davon) von der Montagereihe freisetzt oder abkoppelt. Das Molekül kann beispielsweise durch Hydrolyse oder Zyklisierung freigesetzt werden. Abschlussmodule können eine TE (Thioesterase) -, Cterm- (terminale C-Domäne) oder Re-Domäne umfassen. Das Terminationsmodul befindet sich vorzugsweise am Carboxyterminus eines Polypeptids eines NRPS oder PKS. Das Terminationsmodul kann ferner zusätzliche enzymatische Aktivitäten (z. B. Oligomeraseaktivität) umfassen.

Mit "Domäne" ist eine Polypeptidsequenz oder ein Fragment einer größeren Polypeptidsequenz mit einer oder mehreren spezifischen enzymatischen Aktivitäten gemeint (d. H. C / E-Domänen haben eine C- und eine E-Funktion in einer Domäne oder eine andere konservierte Funktion (d. H. Als Anbindungsfunktion für eine ACP- oder T-Domäne). Somit kann ein einzelnes Polypeptid mehrere Domänen umfassen. Mehrere Domänen können Module bilden. Beispiele für Domänen sind C (Kondensation), Cy (Heterocyclisierung), A (Adenylierung), T (Thiolierung), TE (Thioesterase), E (Epimerisierung), C / E (Kondensation / Epimerisierung), MT (Methyltransferase). Ox (Oxidase), Re (Reduktase), KS (Ketosynthase), AT (Acyltransferase), KR (Ketoreduktase), DH (Dehydratase) und ER (Enoylreduktase).

Mit "nicht ribosomal synthetisiertem Peptid", "nicht ribosomalem Peptid" oder "NRP" ist jedes Polypeptid gemeint, das nicht von einem Ribosom produziert wird. NRPs können linear, zyklisch oder verzweigt sein, und proteinogene, natürliche oder nicht natürliche Aminosäuren, oder eine beliebige Kombination dieser, enthalten. Zu den NRP gehören Peptide, die in einer Art Fließband oder Montagereihe hergestellt werden (= modularer Charakter des Enzymsystems, das die schrittweise Zugabe von Bausteinen zum Endprodukt ermöglicht).

Mit "Polyketid" ist eine Verbindung gemeint, die mehrere Ketyleinheiten umfasst.

Mit "nicht-ribosomaler Peptidsynthetase" oder "nicht-ribosomaler Peptidsynthase" oder "NRPS" ist ein Polypeptid oder eine Reihe von wechselwirkenden Polypeptiden gemeint, die ein nicht-ribosomales Peptid produzieren und somit die Bildung von Peptidbindungen ohne ribosomale Komponenten katalysieren können. Mit "Polyketidsynthase" (PKS) ist ein Polypeptid oder eine Reihe von Polypeptiden gemeint, die ein Polyketid ohne ribosomale Komponenten produzieren.

Mit "nicht-ribosomaler Peptidsynthetase / Polyketidsynthase-Hybrid" oder "Hybrid aus nicht-ribosomaler Peptidsynthetase und Polyketidsynthase" oder "NRPS / PKS-Hybrid" oder "Hybrid aus NRPS und PKS" oder "Hybrid aus PKS und NRPS" und anderen korrespondierenden Wendungen ist ein Enzymsystem gemeint, welches beliebige Domänen oder Module aus NRPS und PKS umfassen. Solche Hybride katalysieren die Synthese von hybriden Naturstoffen.

Mit "Veränderung einer Struktur" ist jede Veränderung einer chemischen (z. B. kovalenten oder nichtkovalenten) Bindung im Vergleich zu einer Referenzstruktur gemeint.

Mit "Mutation" ist eine Veränderung der Nukleinsäuresequenz gemeint, so dass die von der Nukleinsäuresequenz kodierte Aminosäuresequenz mindestens eine Aminosäureveränderung im Vergleich zu der natürlich vorkommenden Sequenz aufweist. Die Mutation kann, ohne Einschränkung, eine Insertions-, Deletions-, Frameshift-Mutation oder eine Missense-Mutation sein. Dieser Begriff beschreibt auch ein Protein, das von der mutierten Nukleinsäuresequenz kodiert wird.

Eine "Variante" ist ein Polypeptid oder Polynukleotid mit mindestens 10%, 20%, 30%, 40%, 50%, 60%, 70%, 80%, 90%, 95% oder 99% Sequenzidentität zu einer Referenzsequenz. Die Sequenzidentität wird typischerweise unter Verwendung einer Sequenzanalysesoftware gemessen (zum Beispiel Sequenzanalyse-Softwarepaket der Genetics Computer Group, Biotechnologiezentrum der Universität Wisconsin, 1710 University Avenue, Madison, Wisconsin, USA). 53705, Programme: BLAST, BESTFIT, GAP oder PILEUP / PRETTYBOX). Solche Software passt identische oder ähnliche Sequenzen an, indem sie verschiedenen Substitutionen, Deletionen und / oder anderen Modifikationen Homologiegrade zuordnet (Substitutions- / Scoring-Matrize: z.B. PAM, Blosum, GONET, JTT).

In einem **ersten Aspekt** betrifft die Erfindung ein Protein oder ein Proteinfragment, umfassend mindestens eine erste Domäne oder Teildomäne einer Nicht-Ribosomalen-Peptidsynthetase (NRPS), einer Polyketidsynthase (PKS) oder einer NRPS/PKS-Hybridsynth(et)ase (erste PKS-NRPS-Domäne), wobei das Protein oder das Proteinfragment einen N-Terminus oder einen C-Terminus umfassend eine erste Bindedomäne aufweist und wobei diese erste Bindedomäne vorzugsweise den N-Terminus, respektive C-Terminus, des Proteins oder des Proteinfragments darstellt, und wobei die erste Bindedomäne durch die Eigenschaft gekennzeichnet ist eine spezifische Protein-Protein Bindung mit mindestens einer korrespondierenden zweiten Bindedomäne eingehen zu können.

Ein Protein oder Proteinfragment, im Sinne dieser Offenbarung, ist vorzugsweise eine Polypeptid umfassend einer Aminosäuresequenz, die eine hohe Sequenzidentität zu einem zusammenhängenden Teilstück einer NRPS/PKS, sowie einer oder mehrere Module dieser, sowie Proteinteile dieser, aufweist.

Der Begriff "Bindedomäne" soll in Kontext dieser Anmeldung ein Polypeptid-Element, Domäne oder Sequenz bezeichnen, die die Fähigkeit aufweist spezifische oder unspezifische kovalente oder nicht-kovalente Bindungen mit anderen Polypeptid-Sequenzen einzugehen. In einer besonderen Ausführungsform handelt es sich bei der zweiten Bindedomäne um eine endogene NRPS/PKS Sequenz, welche eine Wechselwirkung, beispielsweise und vorzugsweise mit einer SYNZIP gemäß der Erfindung ermöglicht. Bevorzugt im Rahmen der vorliegenden Erfindung sind Bindedomänen, die eine spezifische Binde-Wechselwirkung mit anderen korrespondierenden Bindedomänen eingehen. Diese werden in Kontext der vorliegenden Erfindung auch als Proteininteraktionsdomänen (PID) bezeichnet. Beispielsweise können dies Polypeptiddomänen sein, die für die Homo- oder Heterodimerbildung von Protein verantwortlich sind. Solche Domänen sind beispielsweise Coiled-Coil Domänen, CH3 Domänen und Leucin-Zipper Domänen. Insbesondere bevorzugt sind die sogenannten SYNZIP Domänen.

Coiled-Coil-Protein-Interaktionsdomänen sind im Stand der Technik bekannt. Mehrere nicht einschränkende Ausführungsformen von Computerprogrammen zur Erstellung von solchen PIDs umfassen SOCKET (z. B. wie in Walshaw & Woolfson, J. Mol. Gen. Biol, 2001; 307 (5), 1427-1450, erhältlich auf der Website der Woolfson Group an der University of Bristol), COILS (z. B. wie in Lupas et al., Science. 1991; 252: 1162-1164 beschrieben und durch Bezugnahme in diese Offenbarung aufgenommen), erhältlich bei der ch. EMBnet.org-Website), PAIRCOIL (z. B. wie in Berger et al., Proc Natl. Acad. Sci. VEREINIGTE STAATEN VON AMERIKA. 1995; 92, 8259-8263, bei den Gruppen erhältlich. csail.mit.edu/cb/paircoil/cgi-bin/paircoil.cgi und MULTICOIL (z. B. beschrieben von Wolf et al., Protein Sci. 1997; 6: 1179-1189, erhältlich bei der Gruppe s. csail .mit. edu / cb / multicoil / cgi-bin / multicoil cgi-Website.

In einigen Ausführungsformen sind die PIDs, die Coiled-Coils bilden, diejenigen, die in Tabelle I von Müller et al., Methods Enzymol. 2000; 328, 261, welche in dieser Offenbarung in ihrer Gesamtheit durch Bezugnahme aufgenommen sind. Beispielsweise umfassen PIDs, die Coiled-Coils bilden, Leuzinzipper (z. B. wie in den Proteinen GCN4, Fos, Jun, C / EBP und Varianten oder Mutanten davon), das Peptid "Velcro" (z. B. wie von O'Shea et al., Curr Biol. 1993;3(10): 658-67), E-Coil / K-Coil (z. B. wie von Tripet et al., Protein Eng. 1996; 9, 1029 beschrieben) und WinZip-A2 und WinZip-Bl (z. B. wie von Arndt et al. beschrieben), Strukture. 2002; (9): 1235-48).

In einigen Ausführungsformen sind die PIDs, die Coiled-Coils bilden, heterospezifische synthetische Coiled-Coil-Peptide, die SYNZIPs genannt werden, zum Beispiel SYNZIPs 1-22. Detaillierte Informationen zu den SYNZIPs 1-22 sind Thompson KE, et al: "SYNZIP protein interaction toolbox: in vitro and in vivo specifications of heterospecific coiled-coil interaction domains." (ACS Synth Biol. 2012 Apr 20;1(4):118-29.) offenbart; das Dokument ist durch Bezugnahme in seiner Gesamtheit in dieser Offenbarung aufgenommen. In einigen Ausführungsformen sind die PIDs, die entweder C oder N terminal mit einer NRPS-PKS Domäne oder Teildomäne fusioniert sind, SYNZIP 17 (NEKEELKSKKAELRNRIEQLKQKREQLKQKIANLRKEIEAYK, SEQ ID NO: 1) und / oder SYNZIP 18 (SIAATLENDLARLENARLEKDIANLAKLEREEAYEAYEAYEF, SEQ ID NO: 2). Andere kombinationen von SYNZIPs, die in Kontext der vorliegenden Erfindung als Bindedomänenpaar eingesetzt werden können sind in der Matrix der Abbildung 1 aufgeführt.

In einigen Ausführungsformen umfassen die PIDs, die von der vorliegenden Offenbarung in Betracht gezogen werden, diejenigen, die auf der Website von Dr. Tony Pawson im Mount Sinai Hospital, Toronto, offenbart sind. Beispielsweise umfassen PIDs 14-3-3-Domänen, ADF-Domänen, ANK-Wiederholungen, ARM-Wiederholungen, die BAR-Domäne von Amphiphysin, die BEACH-Domäne, Bcl-2-Homologie-Domänen (BH) (z. B. BH1), BH2, BH3, BH4), BIR-Domänen, BRCT-Domänen, Bromodomänen, BTB / POZ-Domänen, CI-Domänen, C2-Domänen, Caspase-Rekrutierungsdomänen (CARDs), lymphoide Myeloid-(CALM-) Domänen mit Clathrin-Assemblierung, Calponin-Homologie (CH-) Domänen, Chromatin Organisationsmodifikator (CHROMO / Chr) Domänen, CUE Domänen, Death (DD) Domänen, Death-Effector (DED) Domänen, DEP Domänen, Dbl Homologie (DH) Domänen, EF-Hand (EFh) Domänen, Epsl5 Homologie (EH) Domänen, epsin NH2-terminale Homologie (ENTH) Domänen, Ena / Vasp-Homologiedomäne 1 (EVH1-Domänen), F-Box-Domänen, FERM-Domänen, FF-Domänen, Formin-Homologiedomänen 2 (FH2), Forkhead-assoziierte Domänen (FH), FYVE (Fab-1, YGL023-, Vps27- und EEA1-Domänen, GAT- (GGA- und Toml-) Domänen, Gelsolin / Severin / Villin-Homologie- (GEL-) Domänen, GLUE- (aus GRAM-ähnlichen Ubiquitin-Bindungsdomänen in EAP45-) Domänen, GRAM- (aus Glucosyltransferasen, Rab-ähnliche Domänen GTPase-Aktivatoren und Myotubularin-Domänen, GRIP-Domänen, Glycin-Tyrosin-Phenylalanin-Domänen (GYF), HEAT-Domänen (von Huntington, Elongationsfaktor 3, PR65 / A, TOR), HECT-Domänen (von homolog zu den E6-AP-Carboxylterminus-Domänen), IQ-Domänen, LIM-Domänen, leucinreiche Repeat-Domänen (LRR-Domänen), maligne Gehirntumor-Domänen (MBT-Domänen), Mad Homology 1-Domänen (MH1-Domänen), MH2-Domänen, MIU-Domänen (von Motif Interacting with Ubiquitin), NZF-Domänen (Npl4-Zinkfinger)) Domänen, PAS-Domänen (Per-ARNT-Sim-Domänen), Phox- und Beml-Domänen (PB1-Domänen), PDZ-Domänen (aus postsynaptischer Dichte 95, PSD-85; große Scheiben, Dig; Zonula occludens-1, ZO-1) ns, Pleckstrin-Homologie-Domänen (PH-Domänen), Polo-Box-Domänen, Phosphotyrosin-Bindungsdomänen (PTB-Domänen), Pumilio-Domänen (Puf-Domänen), PWWP-Domänen, Phox-Homologie-Domänen (PX-Domänen), RGS-Domänen (Regulator of G Protein Signaling), RING-Finger Domänen, SAM-Domänen (steriles Alpha-Motiv), Schatten-Chromo-Domänen (CSD- oder SC-Domänen), Src-Homologie-2-Domänen (SH2-Domänen), Src-Homologie-3-Domänen (SH3-Domänen), SOCS-Domänen (von Suppressoren des Cytokin-Signalwegs), SPRY-Domänen , START (von steroidogenen akuten regulatorischen Protein- (StAR-) verwandten Lipidtransfer-) Domänen, SWIRM-Domänen, Toll / 11-1-Rezeptor- (TIR-) Domänen, Tetratricopeptid-Repeat- (TPR-) Motivdomänen, TRAF-Domänen, SNARE-Domänen (von löslichen NSF-Bindungsprotein-Rezeptoren (SNAP-Rezeptoren)) (z. B. T-SNARE), Tubby-Domänen, Tudor-Domänen, Ubiquitin-assoziierte Domänen (UBA), UEV-Domänen (Ubiquitin E2-Variante), Ubiquitinwechselwirkendes Motiv (UIM) Domänen, Beta-Domänen des von Hippel-Lindau-Tumorsuppressorproteins (VHLP), VHS-Domänen (von Vps27p, Hrs und STAM), WD40-Wiederholungsdomänen und WW-Domänen.

PIDs können mit oder ohne Linker mit C oder N Terminus des Proteins oder Proteinfragments gemäß der Erfindung verbunden werden. Es versteht sich, dass beliebige PIDs und beliebige Linker mit Aspekten der Erfindung kompatibel sein können. In einigen Ausführungsformen ist der Linker flexibel. Der Linker kann aus Aminosäuren zusammengesetzt sein. In einigen Ausführungsformen besteht der Linker aus 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 , 47, 48, 49, 50 oder mehr als 50 Aminosäuren. In einigen Ausführungsformen besteht der Linker aus 5-7 Aminosäuren. In einigen Ausführungsformen ist der Linker beispielsweise ein Gly-Ser-Linker.

Das Protein oder Proteinfragment gemäß der Erfindung umfasst bevorzugt ein SYNZIP als erste und/oder zweite Bindedomäne, wobei das SYNZIP aus einem SYNZIP 1-23 ausgewählt ist, vorzugsweise aus SYNZIP 1, 2, 17, 18 oder 19. Bevorzugt sind in einigen Ausführungsformen ein Protein oder Proteinfragment, wobei der Terminus, der der ersten Bindedomäne gegenüberliegt, eine dritte Bindedomäne umfasst, und wobei die dritte Bindedomäne durch die Eigenschaft gekennzeichnet ist eine spezifische Protein-Protein Bindung mit mindestens einer korrespondierenden vierten Bindedomäne eingehen zu können. Vorzugsweise, wobei die erste und zweite Bindedomäne keine Bindung mit der dritten und vierten Bindedomäne eingehen können. Mit einem solchen Aufbau können erfindungsgemäß NRPS/PKS Proteine erstellt werden, die aus drei oder mehr einzelnen Polypeptiden (Proteine oder Proteinfragmente gemäß der Erfindung) bestehen.

Auch bevorzugt ist, dass die erste und zweite Bindedomäne gezielt eine Bindung mit der dritten oder vierten Bindedomäne eingehen können, so dass Mischungen von Peptiden entstehen können.

Der Begriff "Terminus" in Zusammenhang mit einem Protein oder Proteinteilstück, bezeichnet das jeweilige Ende des Aminosäurenpolymers. Das freie Aminoende wird dabei als "N-Terminus", das freie Carboxy-Ende als "C-Terminus" bezeichnet. Wenn ein Merkmal, beispielsweise eine Sequenz oder Domäne oder andere Elemente, am N- oder C-Terminus angeordnet sind bedeutet dies, dass das entsprechende Merkmal den letzten N-terminalen oder C-terminalen Anteil des gesamten Proteins ausmachen, und somit den N- oder C-Terminus darstellen.

Das Protein oder Proteinfragment gemäß der Erfindung in einer besonderen Ausführungsform umfasst weiter mindestens eine, vorzugsweise zwei, drei oder vier oder mehr, weitere PKS und/oder NRPS-Domäne(n), wobei die weitere(n) PKS und/oder NRPS - Domäne(n) in direkter funktioneller Anordnung neben der ersten PKS-NRPS-Domäne angeordnet ist (sind). Mit direkter funktioneller Anordnung ist gemeint, dass die Domänen räumlich eine NRPS/PKS Synthese eines Peptids oder eines Peptid/Polyketids durchführen können.

In einer bevorzugten Ausführungsform umfasst das Protein oder Proteinfragment nicht die mindestens eine korrespondierende zweite Bindedomäne umfasst. In dieser Ausführungsform befindet sich die zweite Bindedomäne in einem zweiten Protein oder Proteinfragment gemäß der Erfindung, wobei das erste und zweite, vorzugsweise auch weitere, Protein oder Proteinfragment gemäß der Erfindung bilden dann eine Gruppe von erfinderischen Proteinen oder Proteinfragmenten. Diese Gruppe von Proteinen oder Proteinfragmenten gemäß der Erfinder sind derart gestaltet, dass durch spezifische oder unspezifische Bindung mittels der Bindedomänen eine funktionelle NRPS oder PKS, oder Hybride davon, post-translational zusammengesetzt werden können.

In einer bevorzugten Ausführungsform der Erfindung ist die erste Bindedomäne am terminalen Ende derart angeordnet, dass unter normalen Bedingungen eine spezifische oder unspezifische vermittelte Interaktion/Bindung an eine korrespondierende zweite Bindedomäne möglich ist. Hierbei wäre die zweite Bindedomäne in einem erfindungsgemäßen zweiten Protein oder Proteinfragment zu finden. Hierbei würde sich die Zusammensetzung aus NRPS/PKS Domänen in dem ersten und zweiten Protein oder Proteinfragment der Erfindung unterscheiden.

Die erste PKS-NRPS-Domäne, oder Teildomäne, gemäß der Erfindung wird aus einer beliebigen, dem Fachmann bekannten, NRPS und/oder PKS Domäne ausgesucht. Diese sind vorzugsweise auszuwählen aus einer A-Domäne, einer C-Domäne, einer C/E-Domäne, einer E Domäne, einer Cₛₜₐᵣₜ-Domäne, einer FT-Domäne, oder einer T-Domäne. In bevorzugten Ausführungsformen umfasst das Protein oder Proteinfragment der Erfindung mindestens eine A-Domäne, eine C-Domäne und eine T-Domäne, vorzugsweise wobei das Protein oder Proteinfragment mindestens ein NRPS-PKS, Initiierungsmodul, Elongationsmodul, oder Terminationsmodul aufweist.

Das Protein oder Proteinfragment nach Anspruch 12 oder 13, wobei die dritte Bindedomäne über eine Linker-Sequenz an das Protein oder das Proteinfragment gekoppelt ist.

Das Protein oder Proteinfragment nach einem der vorstehenden Ansprüche, wobei die Bindung der ersten Bindedomäne an die zweite Bindedomäne eine nicht-kovalente Bindung ist.

In einem **zweiten Aspekt** betrifft die Erfindung ein isoliertes Nukleinsäurekonstrukt, umfassend einen ersten kodierenden Bereich, der eine Nukleinsäuresequenz aufweist, die für ein Protein oder Proteinfragment des ersten Aspekts kodiert.

Der Begriff "Nukleinsäure" bedeutet natürliche, halbsynthetische oder vollständig synthetische sowie modifizierte Nukleinsäuremoleküle bestehend aus Desoxyribonukleotiden und/oder Ribonukleotiden, und/oder modifizierten Nukleotiden wie beispielsweise "Peptide nucleic acids" (PNA), "locked nucleic acids" (LNA) oder "Phosphorothioaten". Auch andere Modifikationen der Internukleotid-Phosphate und der Ribose bzw. Zucker- Komponenten können enthalten sein.

Ein sogenannter "kodierender Bereich" bezeichnet ein Sequenzelement innerhalb eines Nukleinsäurekonstrukts der Erfindung welches gemäß des genetischen Codes für ein exprimierbares Protein kodiert.

In einer Ausführungsform des Nukleinsäurekonstrukts der Erfindung ist der erste kodierende Bereich funktionell mit einem Expressionspromoter verbunden. Ein Expressionspromoter bezeichnet ein Nukleinsäureelement, welches zur Initiation einer RNA Transkription notwendig, und vorzugsweise hinreichend ist. Solche Elemente sind dem Fachmann bekannt. Je nach Expressionssystem können solche Promotoren ausgewählt werden.

In einer weiteren Ausführungsform umfassent das Nukleinsäurekonstrukt der Erfindung einen zweiten kodierenden Bereich, der eine Nukleinsäuresequenz aufweist, die für ein Protein oder Proteinfragment gemäß der Erfindung kodiert, und wobei der erste kodierende Bereich und der zweite kodierende Bereich für nicht identische Proteine oder Proteinfragmente gemäß der Erfindung kodieren.

In einer weiteren Ausführungsform kann das Nukleinsäurekonstrukt der Erfindung ein oder mehrere weitere Elemente für eine rekombinante Expression, oder Kontrolle der Expressionsstärke oder Zeitpunkts, des Proteins oder Proteinfragments umfassen.

In einem **dritten Aspekt** betrifft die Erfindung ein Vektorsystem zur Herstellung einer funktionellen NRPS oder PKS, oder eines NRPS-PKS-Hybrids, wobei das Vektorsytem mindestens ein Nukleinsäurekonstrukt gemäß des zweiten Aspekts umfasst, und wobei das mindestens eine Nukleinsäurekonstrukt geeignet ist mindestens zwei Proteine oder Proteinfragmente gemäß des ersten Aspekts zu exprimieren, und wobei die mindestens zwei Proteine oder Proteinfragmente unterschiedlich sind und zusammen eine funktionelle NRPS, PKS oder ein NRPS/PKS-Hybrid bilden. Somit können die mindestens zwei Proteine oder Proteinfragmente über ein oder zwei Nukleinsäurekonstrukte exprimiert werden, bzw. insoweit es sich um drei oder mehr Proteine oder Proteinfragmente gemäß der Erfindung handelt, werden diese von einem, zwei oder drei Nukleinsäurekonstrukte exprimiert. Und so weiter. Das Vektorsystem der Erfindung muss nur in Gesamtheit ausreichende kodierende Bereiche bereitstellen um die gewünschte Anzahlt an Proteinen oder Proteinfragmenten gemäß der Erfindung zu exprimieren.

Eine bevorzugte Ausführungsform des Vektorsystems der Erfindung betrifft ein Vektorsystem wobei die mindestens zwei Proteine oder Proteinfragmente, die über die Nukleinsäurekonstrukte exprimierbar sind, über die Bindung zwischen der ersten und/oder zweiten Bindedomäne eine funktionelle NRPS, PKS oder NRPS/PKS-Hybrid bilden. Das heisst, das Vektorsystem gemäß der Erfindung umfasst zumindest zum Teil, solche exprimierbaren Proteine oder Proteinfragmente, die über die Bindedomänen die Fähigkeit aufweisen eine funktionelle NRPS/PKS zu bilden.

Vorzugsweise kann eine funktionelle NRPS, PKS oder NRPS/PKS-Hybrid ein lineares Peptid, zirkuläres Peptid, lineare Polyketid, zirkuläres Polyketid, lineares Peptid-Polyketid oder zirkuläres Peptid-Polyketid synthetisieren.

In einer weiteren Ausführungsform ist es bevorzugt, dass das Vektorsystem Nukleinsäurekonstrukte umfasst, die geeignet sind für die Expression von mindestens drei oder mehr Proteinen oder Proteinfragmenten gemäß der Erfindung oder wobei mindestens zwei der drei oder mehr Proteinen oder Proteinfragmenten zusammen eine funktionelle NRPS, PKS oder ein NRPS/PKS-Hybrid bilden. Weiter können die mindestens drei Proteine oder Proteinfragmente gemeinsam eine funktionelle NRPS, PKS oder NRPS/PKS-Hybrid bilden, und wobei die funktionelle NRPS oder PKS über Bindung der Proteine oder Proteinfragmente untereinander mittels Bindung einer ersten zu einer zweiten Bindedomäne und Bindung einer dritten zu einer vierten Bindedomäne gebildet wird. Hier kann es weiter bevorzugt sein, dass ein erstes Protein oder Proteinfragment eine terminale erste Bindedomäne und eine gegenüberliegend terminale dritte Bindedomäne aufweist, und wobei ein zweites Protein oder Proteinfragment eine terminale zweite Bindedomäne aufweist, und wobei ein drittes Protein oder Proteinfragment eine terminale vierte Bindedomäne aufweist.

Ein bevorzugtes Vektorsystem der Erfindung ist so gestaltet, dass die Bindedomänen zum Zusammenbau der NRPS/PKS gemäß der Erfindung so angeordnet sind, dass diese zwischen den NRPS/PKS Domänen liegen. Bevorzugte Anordnungen der Bindedomänen, insbesondere der SYNZIPs sind den Beispielen zu entnehmen, und zwar intermediär generalisiert nur die offenbarte Position an denen die SYNZIP eingebaut wurden.

In einem **vierten Aspekt** betrifft die Erfindung Eine Verfahren zur Herstellung einer funktionellen (vollständigen) NRPS oder PKS, oder eines NRPS/PKS Hybrids, umfassend das in-Kontakt bringen von mindestens einem ersten Protein oder Proteinfragment gemäß des ersten Aspekts mit einem zweiten Protein oder Proteinfragment gemäß des ersten Aspekts, wobei das erste Protein oder Proteinfragment eine terminale erste Bindedomäne aufweist, und wobei das zweite Protein oder Proteinfragment anstatt der terminalen ersten Bindedomäne die terminale zweite Bindedomäne aufweist. Das Verfahren kann als weiteren Schritt die rekombinante Expression des ersten und/oder zweiten Proteins oder Proteinfragments mittels mindestens eines Nukleinsäurekonstrukts der Erfindung umfassen.

Die Ausdrücke "der [vorliegende] Erfindung", "gemäß der Erfindung", und dergleichen, wie sie hier verwendet werden, sollen sich auf alle Aspekte, Elemente und Ausführungsformen der beschriebenen und/oder beanspruchten Erfindung beziehen.

Wie in der vorliegenden Offenbarung verwendet, soll der Begriff "umfassend" so ausgelegt werden, dass er sowohl "beinhaltend" als auch "bestehend aus" umfasst, wobei beide Bedeutungen im Speziellen auch beabsichtigt sind, und daher individuell offenbarte Ausführungsformen gemäß der vorliegenden Erfindung darstellen. Der Begriff "und / oder" wird als spezifische Offenbarung von jedem der beiden angegebenen Merkmale oder Komponenten mit oder ohne das andere verstanden. Zum Beispiel ist "A und / oder B" als spezifische Offenbarung von jedem von (i) A, (ii) B und (iii) A und B zu verstehen, so als ob jedes einzeln hierin offenbart wäre. Im Rahmen der vorliegenden Erfindung bezeichnen die Begriffe "ungefähr" und "annähernd" ein Genauigkeitsintervall, das der Fachmann in einem Rahmen versteht, um die technische Wirkung des fraglichen Merkmals noch zu gewährleisten. Wo ein unbestimmter oder bestimmter Artikel verwendet wird, wenn auf ein einzelnes Substantiv Bezug genommen wird, z. "ein/eine/eines", oder "der/die/das", schließt diese Verwendung einen Plural dieses Substantivs ein, sofern nicht ausdrücklich etwas anderes angegeben ist.

Es versteht sich, dass die Anwendung der Lehren der vorliegenden Erfindung auf ein spezifisches Problem oder eine spezifische Umgebung angewendet werden können und, dass die Einbeziehung von Variationen der vorliegenden Erfindung oder von zusätzlichen Merkmalen (wie weiteren Aspekten und Ausführungsformen) innerhalb der Fähigkeiten eines Durchschnittsfachmanns und im Lichte der hierin enthaltenen Lehren liegen.

Sofern der Kontext nichts anderes vorschreibt, sind die Beschreibungen und Definitionen der oben dargelegten Merkmale nicht auf einen bestimmten Aspekt oder eine bestimmte Ausführungsform der Erfindung beschränkt und gelten gleichermaßen für alle Aspekte und Ausführungsformen, die beschrieben werden.

Auf alle hier zitierten Referenzen, Patente und Veröffentlichungen wird hiermit in vollem Umfang Bezug genommen.

In Anbetracht des Vorstehenden ist zu beachten, dass sich die vorliegende Erfindung auch auf die folgenden detaillierten und nummerierten Gegenstände bezieht:
Gegenstand 1: Ein Protein oder ein Proteinfragment, umfassend mindestens eine erste Domäne oder Teildomäne einer Nicht-Ribosomalen-Peptidsynthetase (NRPS), einer Polyketidsynthase (PKS) oder einer NRPS/PKS-Hybridsynth(et)ase (erste PKS-NRPS-Domäne), wobei das Protein oder das Proteinfragment einen N-Terminus oder einen C-Terminus umfassend eine erste Bindedomäne aufweist und wobei diese erste Bindedomäne vorzugsweise den N-Terminus, respektive C-Terminus, des Proteins oder des Proteinfragments darstellt, und wobei die erste Bindedomäne durch die Eigenschaft gekennzeichnet ist eine spezifische Protein-Protein Bindung mit mindestens einer korrespondierenden zweiten Bindedomäne eingehen zu können.
Gegenstand 2: Das Protein oder Proteinfragment nach Gegenstand 1, weiter umfassend mindestens eine, vorzugsweise zwei, drei oder vier oder mehr, weitere PKS-NRPS-Domäne(n), wobei die weitere(n) PKS-NRPS-Domäne(n) in direkter funktioneller Anordnung neben der ersten PKS-NRPS-Domäne angeordnet ist (sind).
Gegenstand 3: Das Protein oder Proteinfragment nach Gegenstand 1 oder 2, wobei das Protein oder Proteinfragment nicht die mindestens eine korrespondierende zweite Bindedomäne umfasst.
Gegenstand 4: Das Protein oder Proteinfragment nach einem der Gegenstände 1 bis 3, wobei die erste Bindedomäne am terminalen Ende derart angeordnet ist, dass unter normalen Bedingungen eine spezifische oder unspezifische vermittelte Interaktion/Bindung an eine korrespondierende zweite Bindedomäne möglich ist.
Gegenstand 5: Das Protein oder Proteinfragment nach einem der Gegenstände 1 bis 4, wobei die erste PKS-NRPS-Domäne, oder Teildomäne, ausgesucht wird aus einer A-Domäne, einer A-MT Domäne einer C-Domäne, einer C/E-Domäne, einer E Domäne, einer Cₛₜₐᵣₜ-Domäne, einer FT-Domäne, oder einer T-Domäne.
Gegenstand 6: Das Protein oder Proteinfragment nach einem der Gegenstände 1 bis 5, umfassend mindestens eine A- oder A-MT Domäne, eine C-Domäne und/oder E-Domäne oder eine C/E-Domäne oder eine Cy-Domäne, und eine T-Domäne, vorzugsweise wobei das Protein oder Proteinfragment mindestens ein NRPS-PKS Elongationsmodul aufweist.
Gegenstand 7: Das Protein oder Proteinfragment nach einem der Gegenstände 1 bis 6, wobei die Bindedomäne eine Proteinsequenz ist, vorzugsweise eine Proteindomäne die eine spezifische Protein-Protein Bindung vermittelt.
Gegenstand 8: Das Protein oder Proteinfragment nach einem der Gegenstände 1 bis 7, wobei die Bindedomäne eine Coiled-coil Domäne umfasst.
Gegenstand 9: Das Protein oder Proteinfragment nach Gegenstand 8, wobei die Bindedomäne eine synthetische Coiled-coil Domäne (SYNZIP) umfasst.
Gegenstand 10: Das Protein oder Proteinfragment nach Gegenstand 9, wobei der SYNZIP aus einem SYNZIP 1-23 ausgewählt ist, vorzugsweise aus SYNZIP 1, 2, 17, 18 oder 19. Das Protein oder Proteinfragment, das der ersten Bindedomäne gegenüberliegenden Terminus eine dritte Bindedomäne umfasst, und wobei die dritte Bindedomäne durch die Eigenschaft gekennzeichnet ist eine spezifische Protein-Protein Bindung mit mindestens einer korrespondierenden vierten Bindedomäne eingehen zu können.
Gegenstand 11: Das Protein oder Proteinfragment nach Gegenstand 11, wobei die erste und zweite Bindedomäne keine Bindung mit der dritten und vierten Bindedomäne eingehen können.
Gegenstand 12: Das Protein oder Proteinfragment nach Gegenstand 11, wobei die erste und zweite Bindedomäne gezielt eine Bindung mit der dritten oder vierten Bindedomäne eingehen können, so dass Mischungen von Peptiden entstehen können.
Gegenstand 13: Das Protein oder Proteinfragment nach einem der Gegenstände 1 bis 12, wobei die erste Bindedomäne durch eine Linker-Sequenz mit der ersten PKS-NRPS-Domäne verbunden ist.
Gegenstand 14: Das Protein oder Proteinfragment nach Gegenstand 12 oder 13, wobei die dritte Bindedomäne über eine Linker-Sequenz an das Protein oder das Proteinfragment gekoppelt ist.
Gegenstand 15: Das Protein oder Proteinfragment nach einem der vorstehenden Gegenstände, wobei die Bindung der ersten Bindedomäne an die zweite Bindedomäne eine nicht-kovalente Bindung ist.
Gegenstand 16: Ein isoliertes Nukleinsäurekonstrukt, umfassend einen ersten kodierenden Bereich, der eine Nukleinsäuresequenz aufweist, die für ein Protein oder Proteinfragment nach einem der Gegenstände 1 bis 15 kodiert.
Gegenstand 17: Das isolierte Nukleinsäurekonstrukt nach Gegenstand 16, wobei der erste kodierende Bereich funktionell mit einem Expressionspromoter verbunden ist.
Gegenstand 18: Das isolierte Nukleinsäurekonstrukt nach Gegenstand 16 oder 17, weiter umfassend einen zweiten kodierenden Bereich, der eine Nukleinsäuresequenz aufweist, die für ein Protein oder Proteinfragment nach einem der Gegenstände 1 bis 15 kodiert, und wobei der erste kodierende Bereich und der zweite kodierende Bereich für nicht identische Proteine oder Proteinfragmente kodieren.
Gegenstand 19: Das isolierte Nukleinsäurekonstrukt nach einem der Gegenstände 1 bis 18, welches weitere Elemente für eine rekombinante Expression des Proteins oder Proteinfragments umfasst.
Gegenstand 20: Ein Vektorsystem zur Herstellung einer funktionellen NRPS oder PKS, wobei das Vektorsystem mindestens ein Nukleinsäurekonstrukt gemäß eines der Gegenstände 16 bis 20 umfasst, und wobei das mindestens ein Nukleinsäurekonstrukt geeignet ist, mindestens zwei Proteine oder Proteinfragmente gemäß einem der Gegenstände 1 bis 15 zu exprimieren, und wobei die mindestens zwei Proteine oder Proteinfragmente unterschiedlich sind und zusammen eine funktionelle NRPS, PKS oder NRPS/PKS-Hybrid bilden.
Gegenstand 21: Das Vektorsystem nach Gegenstand 20, wobei die mindestens zwei Proteine oder Proteinfragmente über die Bindung der ersten und zweiten Bindedomäne die funktionelle NRPS, PKS oder NRPS/PKS-Hybrid bilden.
Gegenstand 22: Das Vektorsystem nach Gegenstand 20 oder 21, wobei eine funktionelle NRPS, PKS oder NRPS/PKS-Hybrid ein lineares Peptid, zirkuläres Peptid, lineare Polyketid, zirkuläres Polyketid, lineares Peptid-Polyketid oder zirkuläres Peptid-Polyketid synthetisieren kann.
Gegenstand 23: Das Vektorsystem nach einem der Gegenstände 20 bis 22, wobei das Vektorsystem Nukleinsäurekonstrukte umfasst, die geeignet sind für die Expression von mindestens drei oder mehr Proteinen oder Proteinfragmenten gemäß eines der Gegenstände 1 bis 15, oder wobei mindestens zwei der drei oder mehr Proteinen oder Proteinfragmenten zusammen eine funktionelle NRPS, PKS oder ein NRPS/PKS-Hybrid bilden.
Gegenstand 24: Das Vektorsystem nach Gegenstand 23, wobei die mindestens drei Proteine oder Proteinfragmente gemeinsam eine funktionelle NRPS, PKS oder NRPS/PKS-Hybrid bilden, und wobei die funktionelle NRPS oder PKS über Bindung der Proteine oder Proteinfragmente untereinander mittels Bindung einer ersten zu einer zweiten Bindedomäne und Bindung einer dritten zu einer vierten Bindedomäne gebildet wird.
Gegenstand 25: Das Vektorsystem nach Gegenstand 23 oder 24, wobei ein erstes Protein oder Proteinfragment eine terminale erste Bindedomäne und eine gegenüberliegend terminale dritte Bindedomäne aufweist, und wobei ein zweites Protein oder Proteinfragment eine terminale zweite Bindedomäne aufweist, und wobei ein drittes Protein oder Proteinfragment eine terminale vierte Bindedomäne aufweist.
Gegenstand 26: Eine Verfahren zur Herstellung einer funktionellen (vollständigen) NRPS oder PKS, umfassend das in-Kontakt bringen von mindestens einem ersten Protein oder Proteinfragment gemäß einem der Gegenstände 1 bis 15 mit einem zweiten Protein oder Proteinfragment gemäß einem der Gegenstände 1 bis 15, wobei das erste Protein oder Proteinfragment eine terminale erste Bindedomäne aufweist, und wobei das zweite Protein oder Proteinfragment anstatt der terminalen ersten Bindedomäne die terminale zweite Bindedomäne aufweist.
Gegenstand 27: Das Verfahren nach Gegenstand 26, wobei das in-Kontakt bringen die rekombinante Expression des ersten und/oder zweiten Proteins oder Proteinfragments mittels mindestens eines Nukleinsäurekonstrukts nach einem der Gegenstände 16 bis 19 umfasst.

### KURZE BESCHREIBUNG DER ABBILDUNGEN UND SEQUENZEN

### Die Abbildungen zeigen

**Abbildung 1****:** zeigt die SYNZIP Interaktionspartner und mögliche Netzwerke. A) Protein-Mikroarray-Assay Ergebnisse von 26 Peptiden, die spezifische Interaktionspaare bilden. Peptide, die auf der Oberfläche des Mikroarrays immobilisiert sind, sind in Reihe gezeigt. Fluoreszenzmarkierte Peptide in Lösung sind in Zeile gelistet. Entsprechend des Arrayscores (rechts angegeben), zeigen schwarze Spots ein starkes (0-0,2) und weiße Spots ein schwaches Fluoreszenzsiganel (>1,0). Die Abwesenheit von homospezifischen Interaktionen ist durch die rote Diagonale angegeben. Interaktionen, die einen Arrayscore von ≤ 0,2 zeigten, sind grün hervorgehoben. Die Anzahl starker Interaktionspartner ist in der unteren Spalte gezeigt (Reinke et al., 2010). B) Mögliche SYNZIP Interaktions-Netzwerke: 1. lineares 2. ringförmiges 3. verzweigtes und 4. orthogonales Netzwerk mit den entsprechenden SYNZIP-Nummern sind angegeben. Gestrichelte Linien zeigen eine schwache und durchgezogene Linien eine starke Interaktion an. Der Stern hebt die antiparallele Interaktion zwischen SYZIP17 und SYNZIP18 hervor (Thompson et al., 2012).

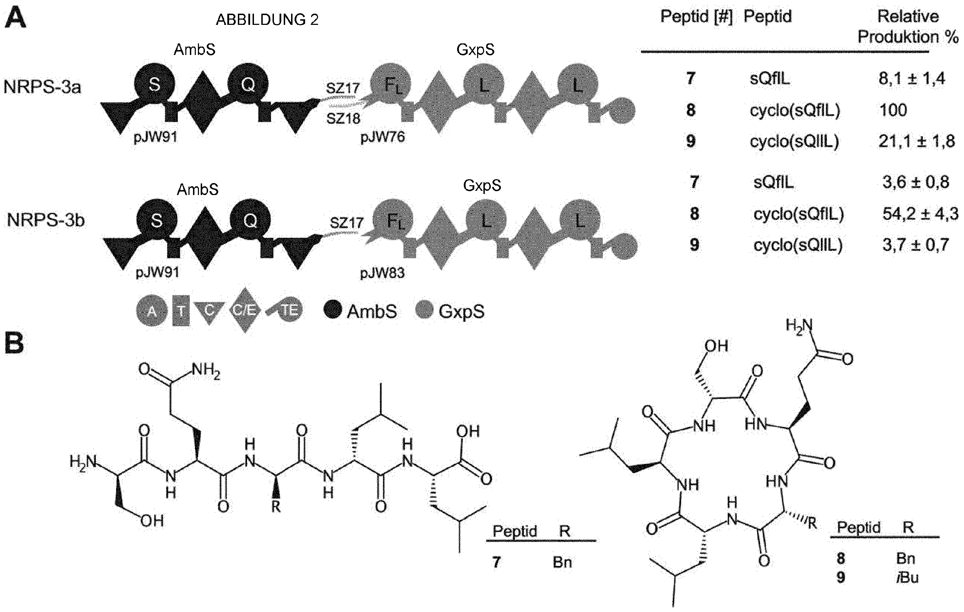

**Abbildung 2****:** zeigt die Konstruktion eines AmbS-Hybriden für die Produktion neuartiger Peptide. A: Schematische Darstellung der NRPS-Hybriden (NRPS-3a undNRPS-3b) aus XUs der AmbS (schwarz) und GxpS (rot). Die dazugehörige relative Peptidproduktion der Peptide 7, 8 und 9 aus Triplikat-Messungen ist in % gezeigt. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18. B: Struktur der produzierten Peptide.

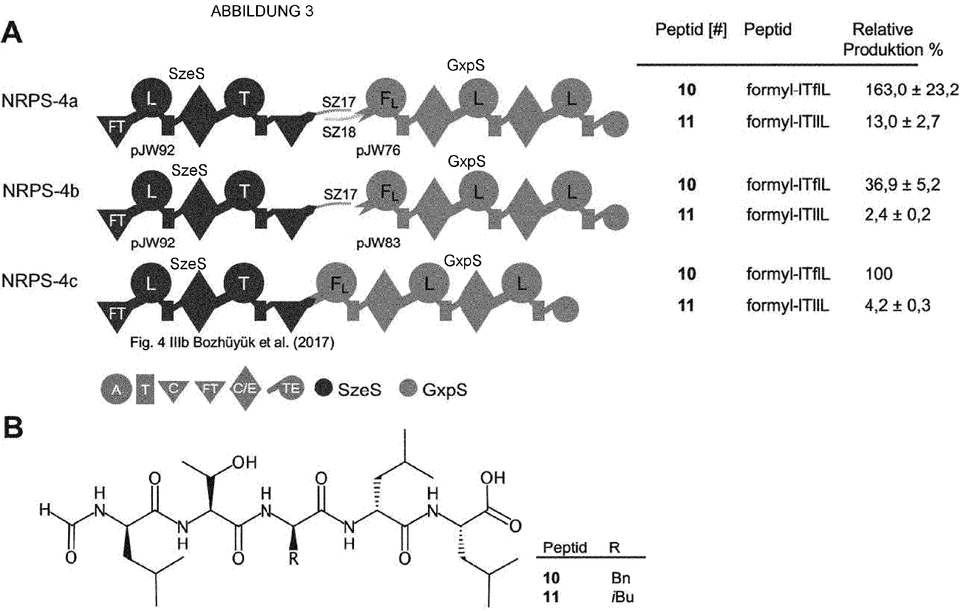

**Abbildung 3****:** zeigt die Konstruktion eines SzeS-Hybriden für die Produktion neuartiger Peptide. **A:** Schematische Darstellung der NRPS-Hybriden (NRPS-4a und NRPS-4b) und des kovalent verknüpften Hybriden (NRPS-4c) aus XUs der SzeS (grün) und GxpS (rot). Die dazugehörige relative Peptidproduktion der Peptide **10** und **11** aus Triplikat-Messungen ist in % gezeigt. Symbole repräsentieren Domänen: Kreis, A-Domäne; Rechteck, T-Domäne; Dreieck, C-Domäne oder FT-Domäne; Raute, C/E-Domäne; kleiner Kreis am C-Terminus, TE-Domäne. Helices repräsentieren SZs: orange, SZ17; grün, SZ18. **B:** Struktur der produzierten Peptide.

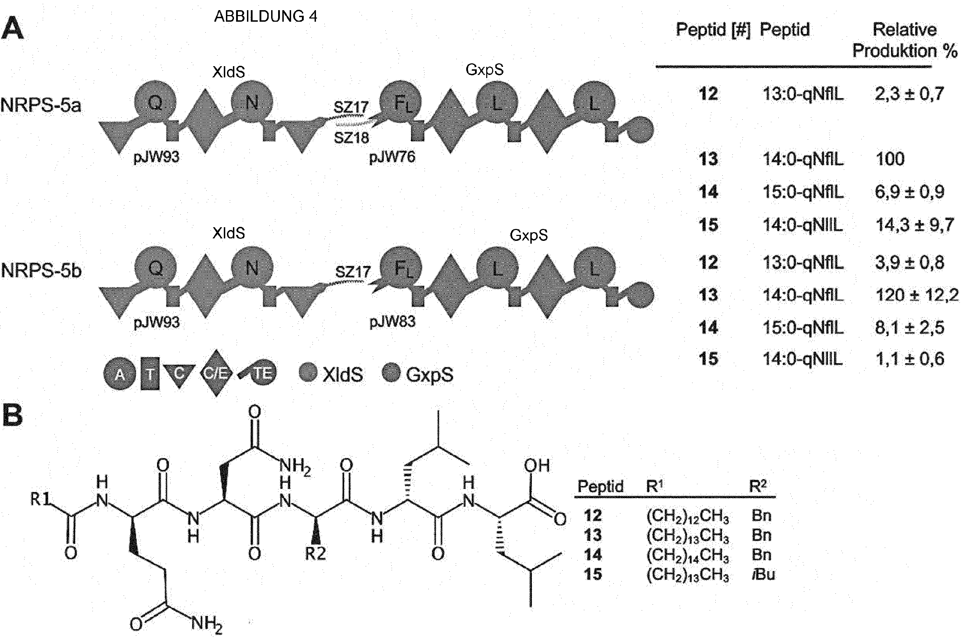

**Abbildung 4****:** zeigt die Konstruktion eines XldS-Hybriden für die Produktion neuartiger Peptide. **A:** Schematische Darstellung der NRPS-Hybriden (NRPS-5a und NRPS-5b) aus XUs der XldS (türkis) und GxpS (rot). Die dazugehörige relative Peptidproduktion der Peptide **12, 13, 14** und **15** aus Triplikat-Messungen ist in % gezeigt. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18. **B:** Struktur der produzierten Peptide.

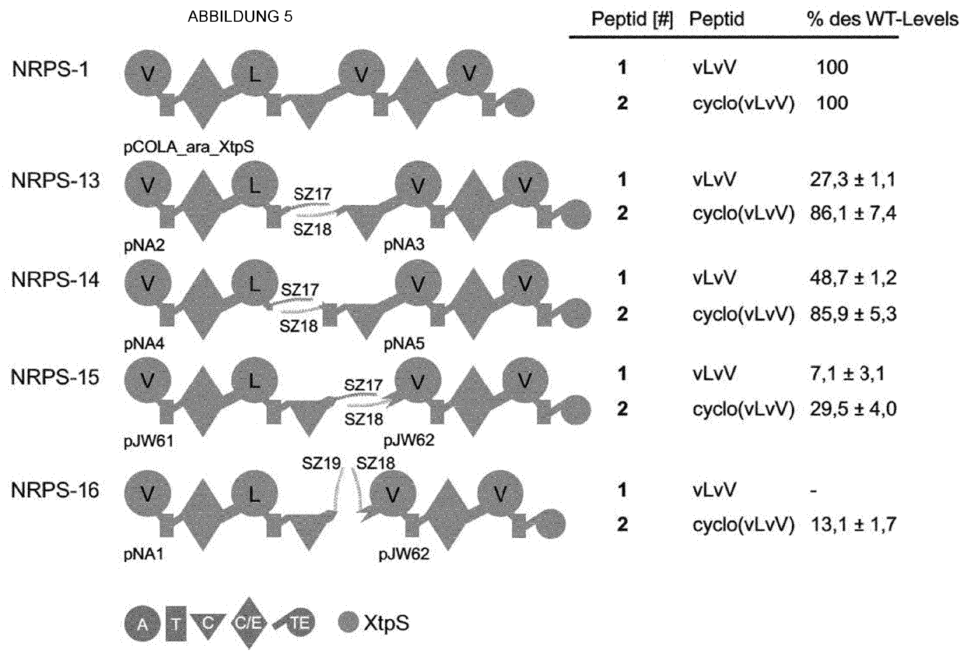

**Abbildung 5****:** zeigt den Konzeptnachweis verschiedener Schnittstellen und SZ-Oligomerisierungsstatus auf Grundlage der XtpS. Schematische Darstellung der im T-C (NRPS-13), A-T (NRPS-14) und C-A (NRPS-15 und NRPS-16) geteilten XtpS (hellgrün) sowie Konstrukte mit unterschiedlichen SZ-Oligomerisierungstatus (NRPS-15 und NRPS-16). Als Referenz wurde die WT-XtpS (NRPS-1) verwendet. Die relative Produktion der Peptide **1** und **2** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18; gelb: SZ19.

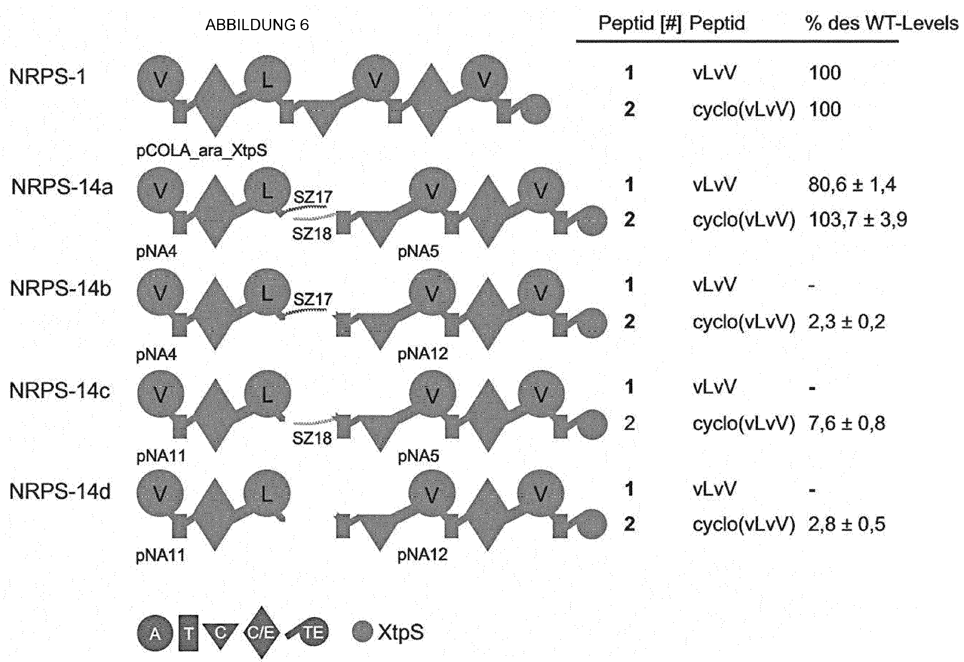

**Abbildung 6****:** zeigt den Einfluss der SZs auf die Produktion der A-T geteilten XtpS. Schematische Darstellung der drei Kontrollexperimente ohne N-terminalen SZ (NRPS-14b), C-terminalen SZ (NRPS-14c) und beiden SZs (NRPS-14d), sowie Darstellung des Konstrukts mit beiden SZs (NRPS-14a). Als Referenz wurde die WT-XtpS (NRPS-1) verwendet. Die relative Produktion der Peptide **1** und **2** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18.

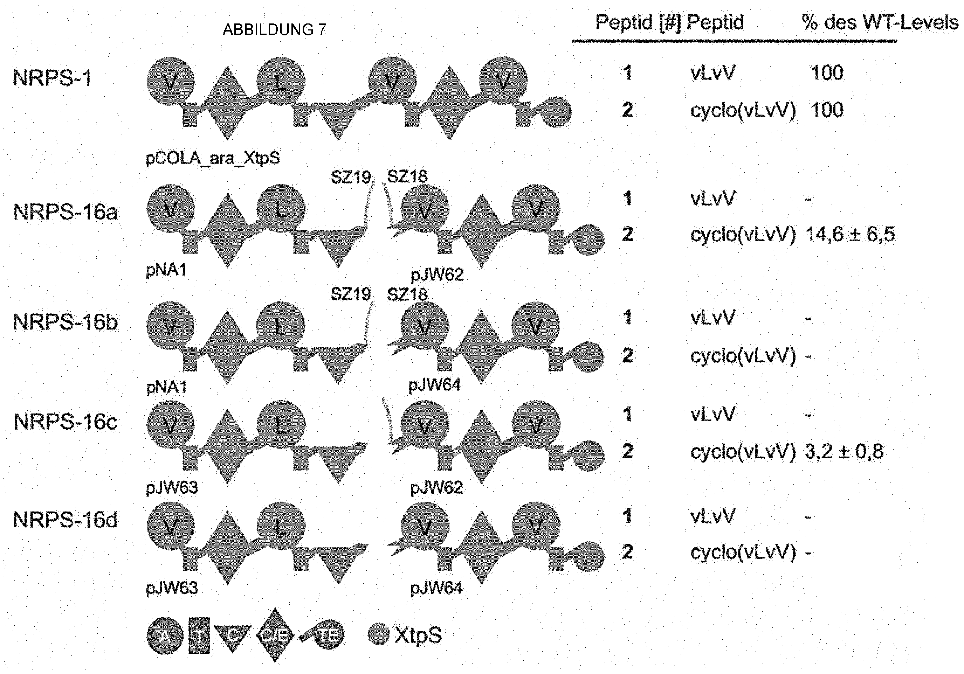

**Abbildung 7****:** zeigt den Einfluss der SZs auf die Produktion der C-A (SZ19/18) geteilten XtpS. Schematische Darstellung der drei Kontrollexperimente ohne N-terminalen SZ (NRPS-16b), C-terminalen SZ (NRPS-16c) und beiden SZs (NRPS-16d), sowie Darstellung des Konstrukts mit beiden SZs (NRPS-16a). Als Referenz wurde die WT-XtpS (NRPS-1) verwendet. Die relative Produktion der Peptide **1** und **2** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: gelb, SZ19; grün, SZ18.

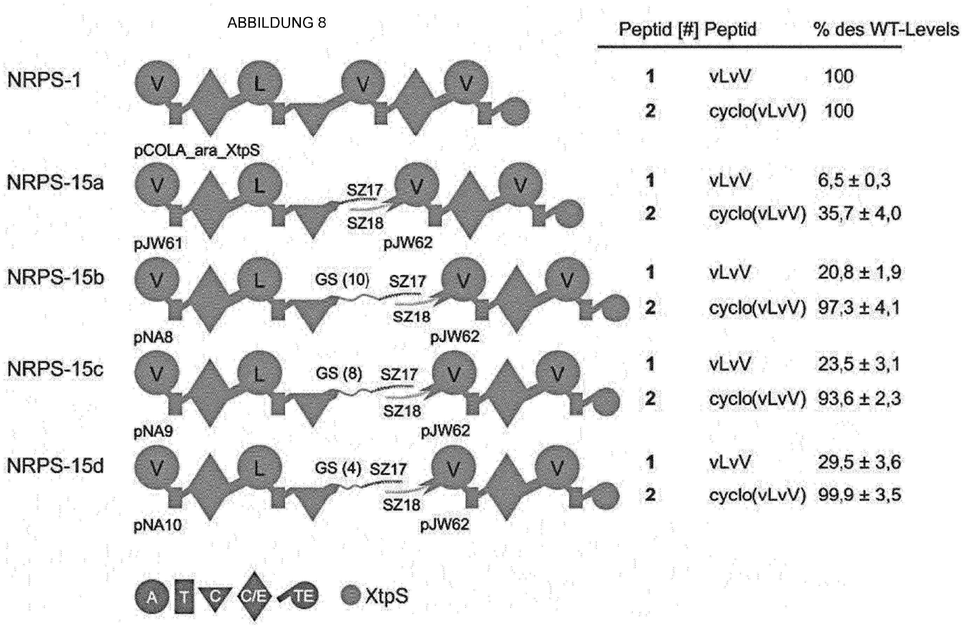

**Abbildung 8****:** zeigt den Einfluss von GS-Linkern auf die Produktion der C-A (SZ17/18) geteilten XtpS. Schematische Darstellung des Konstrukte ohne GS-Linker (NRPS-15a) und mit einem 10 AS langen (NRPS-15b), 8 AS langem (NRPS-15c) und 4 AS langem GS-Linker (NRPS-15d), der zwischen dem C-terminalen Ende des ersten XtpS Abschnitts und SZ 17 eingeführt wurde. Als Referenz wurde die WT-XtpS (NRPS-1) verwendet. Die relative Produktion der Peptide **1** und **2** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18.

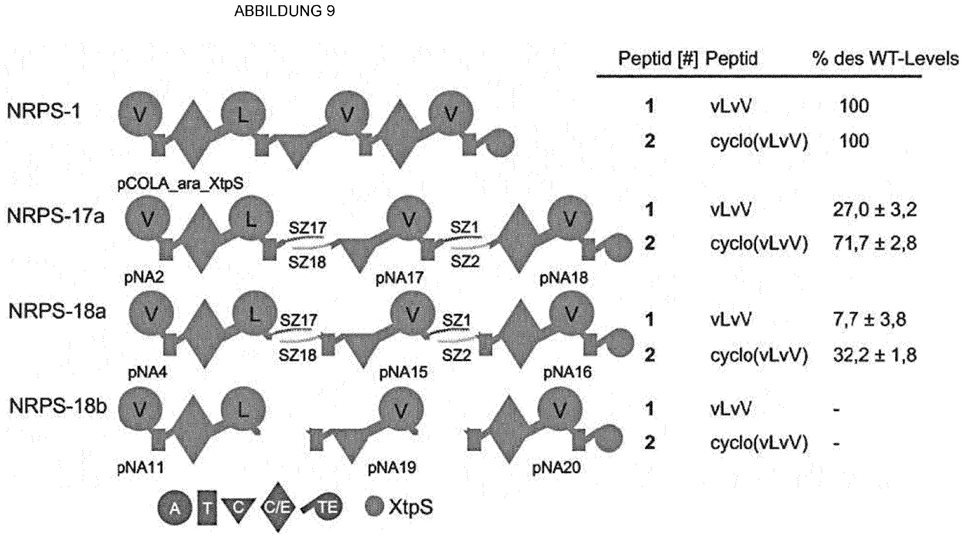

**Abbildung 9****:** zeigt die Produktivität der dreigeteilten XtpS. Schematische Darstellung der in den T-C (NRPS-17a) und A-T (NRPS-18a) Linkern geteilten XtpS und entsprechenden Negativkontrolle (NRPS-18b). Als Referenz wurde die WT-XtpS (NRPS-1) verwendet. Die relative Produktion der Peptide **1** und **2** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2.

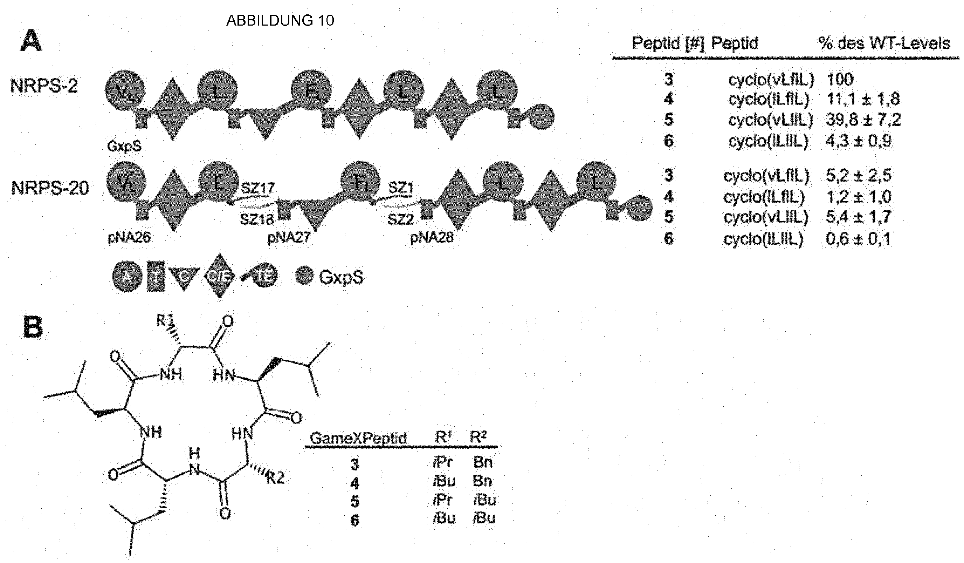

**Abbildung 10****:** zeigt die Produktivität der dreigeteilten GxpS. **A:** Schematische Darstellung der in den A-T Linkern geteilten GxpS (NRPS-20). Als Referenz wurde die WT-GxpS (NRPS-2) verwendet. Die relative Produktion der Peptide **3, 4, 5** und **6** aus Triplikat-Messungen ist in % des WT-Levels angegeben. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2. **B:** Struktur der produzierten Peptide.

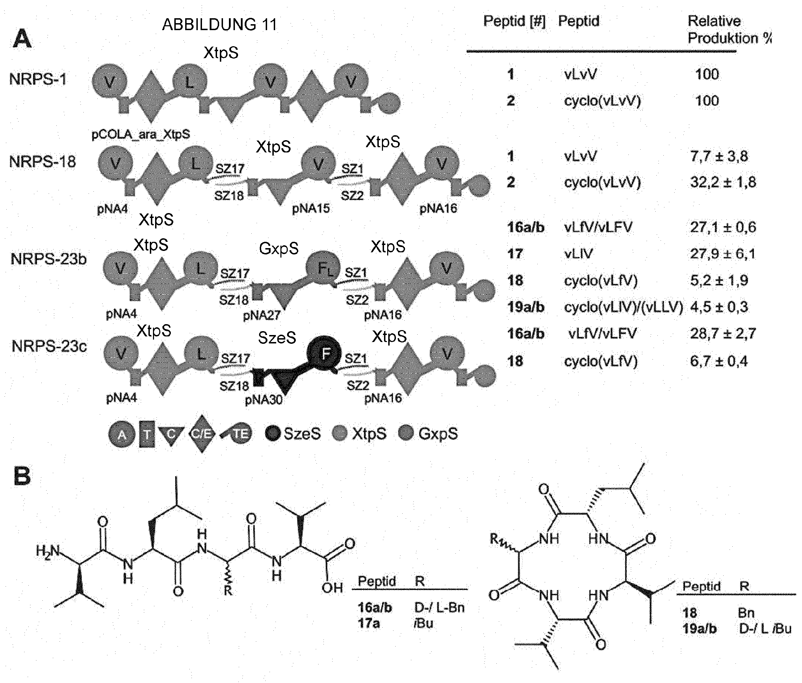

**Abbildung 11****:** zeigt die Reprogrammierung der XtpS für die Produktion neuartiger Peptide. **A:** Schematische Darstellung der Hybriden NRPS-23b und NRPS-23c, die durch die Substitution der XtpS (hellgrün)-Tridomäne gegen eine GxpS (rot) und SzeS (grün) Tridomäne erzeugt wurden. Die relative Produktion der Peptide **16a/b, 17a, 18** und **19a/b** aus Triplikat-Messungen ist in % gezeigt im Vergleich zum WT (NRPS-1) normiert. Die Dreiteilung von XtpS (NRPS-18) ist ebenfalls gezeigt. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2. **B:** Struktur der produzierten Peptide.

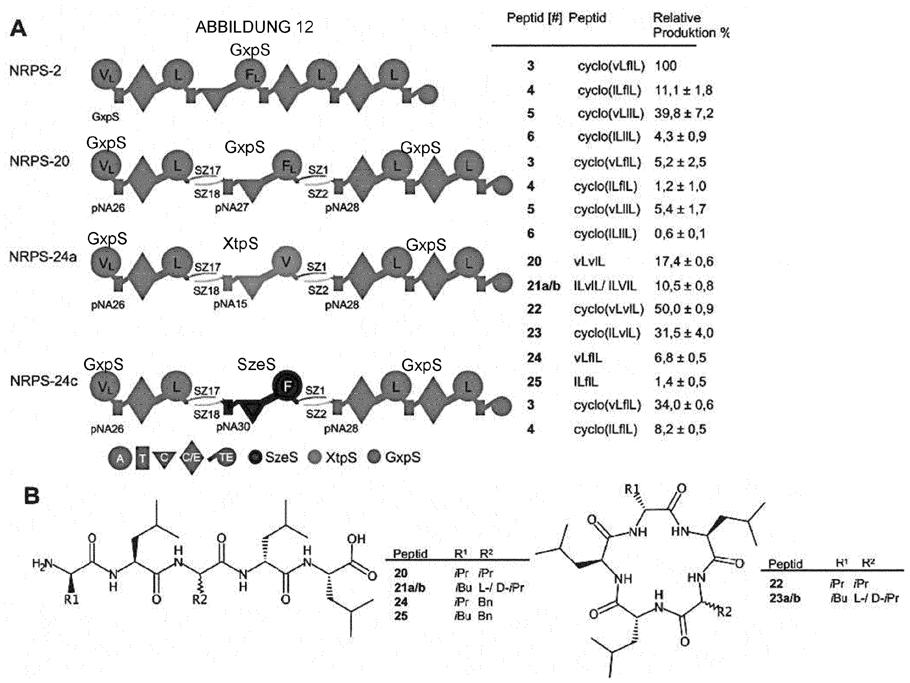

**Abbildung 12****:** zeigt die Reprogrammierung der GxpS für die Produktion neuartiger Peptide. **A:** Schematische Darstellung der Hybriden NRPS-24a und NRPS-24c, die durch die Substitution der GxpS (rot)-Tridomäne gegen eine XtpS, (hellgrün) und SzeS (grün) Tridomäne erzeugt wurden. Die relative Produktion der Peptide **20, 21a/b, 22, 23a/b, 24, 25, 3** und **2** aus Triplikat-Messungen ist in % im Vergleich zum WT (NRPS-2) gezeigt. Die Dreiteilung von GxpS (NRPS-20) ist ebenfalls gezeigt. Symbole repräsentieren Domänen: Kreis, A-Domäne; Rechteck, T-Domäne; Dreieck, C-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2 **B:** Struktur der produzierten Peptide.

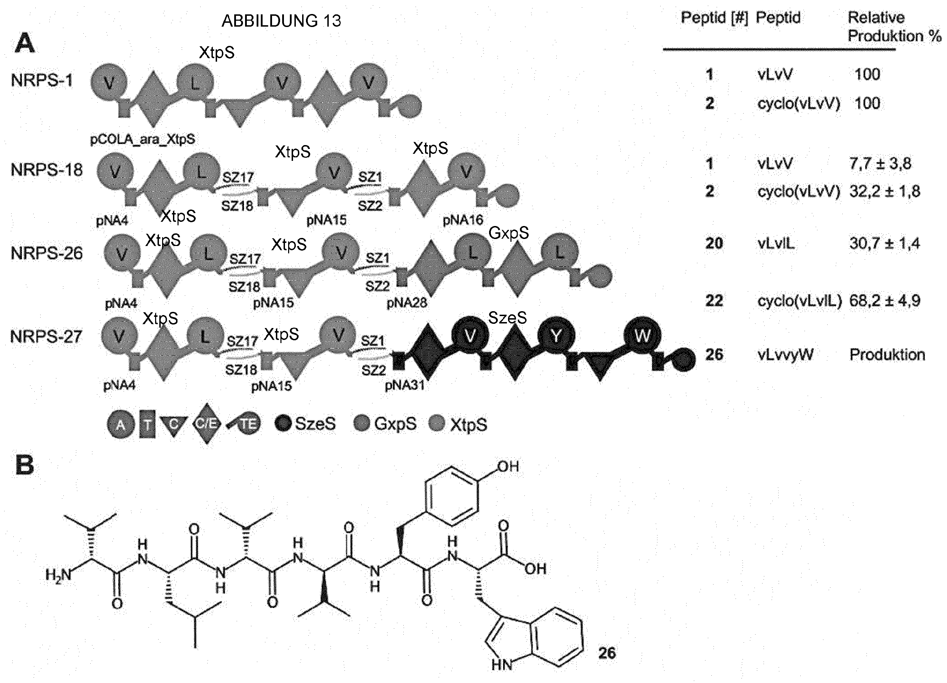

**Abbildung 13****:** zeigt das Design von XtpS-Hybriden für die Produktion neuartiger Peptide. **A:** Schematische Darstellung der Hybriden NRPS-26 und NRPS-27, die jeweils aus Teilen der GxpS (rot) und SzeS (grün) sowie XtpS (hellgrün) erzeugt wurden. Die dazugehörige relative Peptidproduktion der Peptide **20, 22** und **26** aus Triplikat-Messungen ist in % gezeigt. Die Dreiteilung von XtpS (NRPS-18) ist ebenfalls gezeigt. Symbole repräsentieren Domänen: Kreis, A-Domäne; Rechteck, T-Domäne; Dreieck, C-Domäne oder FT-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2. **B:** Struktur der produzierten Peptide.

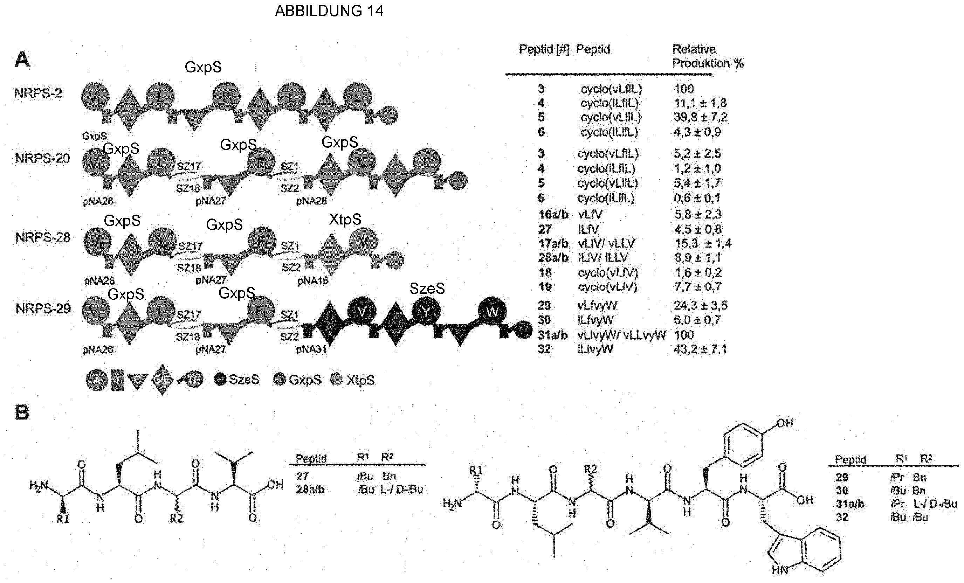

**Abbildung 14****:** zeigt das Design von GxpS-Hybriden für die Produktion neuartiger Peptide. **A:** Schematische Darstellung der Hybriden NRPS-28 und NRPS-29, die jeweils aus Teilen der XtpS (hellgrün) und SzeS (grün) sowie GxpS (rot) erzeugt wurden. Die dazugehörige relative Peptidproduktion der Peptide **16a/b, 27, 17a/b, 28a/b, 18, 19, 29, 30, 31a/b** und **32** aus Triplikat-Messungen ist in % gezeigt. Die Dreiteilung von GxpS (NRPS-20) ist ebenfalls gezeigt. Symbole repräsentieren Domänen: Kreis, A-Domäne; Rechteck, T-Domäne; Dreieck, C-Domäne oder FT-Domäne; Raute, C/E-Domäne; kleiner Kreis am C-Terminus, TE-Domäne. Helices repräsentieren SZs: orange, SZ17; grün, SZ18; dunkelblau: SZ1; hellblau: SZ2. **B:** Struktur der produzierten Peptide.

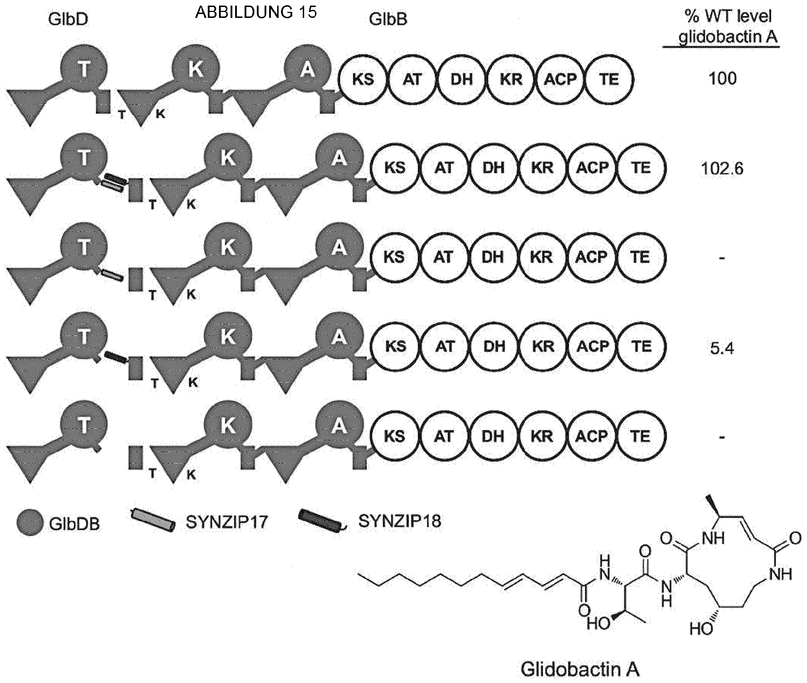

**Abbildung 15****:** zeigt die Teilung eines Hybrids aus NRPS und PKS Modulen. Der durch das Hybrid hergestellte Stoff ist Glidobactin A (siehe Struktur). Die NRPS GlbD wurde zwischen A- und T-Domäne mit SZs geteilt. Symbole repräsentieren Domänen: Kreis, A-Domäne; Rechteck, T-Domäne; Dreieck, C-Domäne; PKS Domänen in GlbB sind entsprechend nach ihren Funktionen benannt. Helices repräsentieren SZs: hellgrau, SZ17; dunkelgrau, SZ18.

### Die Sequenzen zeigen:

**SEQ ID Nrn. 1 und 2:** SYNZIP Sequenzen
**SEQ ID Nrn. 3 und 5:** Bevorzugte Sequenzmotive zur Einfügung der Bindedomänen gemäß der Erfindung
**SEQ ID Nr. 6 bis 30:** Peptidsequenzen der in dieser Anmeldung produzierten NRPS Peptide

### BEISPIELE

Bestimmte Aspekte und Ausführungsformen der Erfindung werden nun beispielhaft und unter Bezugnahme auf die hier dargelegten Beschreibungen, Figuren und Tabellen veranschaulicht. Solche Beispiele der Stoffe, Verfahren, Verwendungen und anderen Aspekte der vorliegenden Erfindung sind nur repräsentativ und sollten nicht einschränkend verstanden werden.

### Die Beispiele zeigen:

### Beispiel 1: SYNZIP vermitteltes de novo Design von NRPSs auf Grundlage des XU-Konzepts

Diese Arbeit befasste sich zunächst mit der *de novo* Konstruktion von NRPSs und der Produktion neuartiger Peptide auf Grundlage des XU-Konzeptes. Unter Einführung des SYNZIP Paars 17/18 in das konservierte WNATE-Motiv des C-A Linkers sollten Hybrid-NRPS aus zwei Systemen konstruiert werden. Das antiparallele SZ-Paar sollte dabei als nicht-kovalenter Mediator zwischen den verschiedenen Synthetasen dienen. Mit einer Dissoziationskonstante (Kd) von < 10 nM weisen SZ17 und SZ18 eine starke Affinität zueinander auf (Thompson et al., 2017), sodass nahezu Eigenschaften einer kovalenten Verknüpfung vorliegen. Durch die Kombination der ersten beiden XUs der AmbS, SzeS und XldS mit den letzten drei XUs der GxpS sollten die NRPS-Hybriden erzeugt werden. SZ17 wurde dafür an das C-Terminal Ende der AmbS, SzeS und XldS und SZ18 an das N-Terminal Ende der GxpS angefügt. In Bezug auf die durch Bozhüyük et al. aufgestellte Regel, die die Berücksichtigung der C-Domänen-Spezifität verlangt, wurden die Spezifitäten für die ersten beiden Hybriden (AmbS-GxpS- und Sze-GxpS), jedoch nicht für den letzten (XldS-GxpS) eingehalten.

### Beispiel 2: Plasmidkonstruktion und heterologe Expression von GxpS-Hybriden in E. coli DH10B::mtaA

Die ersten zwei XU der AmbS (A1-C3), SzeS (C1-C3) und XldS (C1-C3) wurden zunächst anhand der gDNA aus *X. miraniensis* DSM 17902, *X. szentirmaii* DSM 16338 und *X. indica* DSM17382 amplifiziert. Hierzu wurden die in Tab. 3 aufgelisteten Primer verwendet. Diese enthielten passende Überhänge zu einem pACYC_ara_araE Vektor, der bereits die Sequenz des SZ17 enthielt. Nach Linearisierung des Vektors, erfolgte die Klonierung der Plasmide pJW91 (*ambS*_A1-C3-SZ17), pJW92 (*szeS_*C1-C3*_*SZ17), und pJW93 (*xldS_*C1-C3_SZ17) aus Plasmidrückgrat und Inserts durch eine Hot-Fusion-Reaktion (s. 2.3.7). Nach Screening und Verifizierung der Plasmide, wurden diese jeweils mit einem weiteren Plasmid pJW76 (SZ18_*gxpS*_A3-TE) oder pJW83 (*gxpS*_A3-TE) gemeinsam in *E. coli* DH10B::*mtaA* transformiert. pJW76 enthielt dabei die Sequenz der letzten drei XUs der GxpS sowie die Sequenz des SZ18. Die Transformation von pJW83, dem die Sequenz von SZ18 fehlt, diente dagegen als Negativkontrolle. Die Proteinproduktion erfolgte durch Induktion mit L(+)-Arabinose in Triplikate bei 22°C für 72h.

Bei der anschließenden Analyse mittels HPLC-MS (s. 2.5.2) wurde nach den Massen der Peptide gesucht, die sich aus den Hybrid-Systemen ergeben würden. Für den Hybriden NRPS-3a, der sich aus Teilen der AmbS und GxpS zusammensetzte, wurde dementsprechend nach m/z-Werten von 607,23 [M+H⁺] für 7 (lineares Peptid) und 589,33 [M+H⁺] **8** (zyklisches Peptid) gesucht. Diese Massen ließen sich aus der Peptidsequenz (sQflL) errechnen. Aufgrund der Promiskuität der dritten GxpS A-Domäne, die in der Lage ist neben Phenylalanin auch Leucin einzubauen, wurden des Weiteren auch nach m/z-Werten von 573,36 [M+H⁺] (lineares Peptid) und 555,35 [M+H⁺] (**9**) (zyklisches Peptid) gesucht. In diesem Fall ergaben sich die Massen aus der Sequenz (sQllL). Die Peptide **7,8** und **9,** die bei einer Retentionszeit von 6 min, 7,1 min und 7 min eluierten, konnten anhand ihrer Masse identifiziert werden. Das lineare Peptid mit der Sequenz (sQllL) konnte dagegen nicht detektiert werden. Da zum Zeitpunkt der Datenaufnahme kein Standard zu Verfügung stand, erfolgte die Quantifizierung der Ergebnisse relativ und wurde aus dem Mittelwert der *Peak Area* berechnet (Abb. 2). Hieraus ergab sich, dass **8** das am häufigsten detektierte Peptid war. **7** und **9** wurden mit 8,1% und 21,2% relativ zu **8** produziert. Alle Peptide ließen sich anhand ihres MS²-Spektrums verifizieren (Anhang Abb. 2). Des Weiteren ergaben die Messdaten der Negativkontrolle (NRPS-3b), dass die Produktion von **7, 8** und **9** auch ohne N-terminalen SZ möglich ist (Abb. 7). Die Produktion der Peptide war jedoch signifikant geringer als im Vergleichssystem mit beiden SZs (NRPS-3a). So wurden **7** und **8** nur noch zu ∼50% und Peptid **9** nur noch zu ∼18% produziert.

Für den zweiten Hybriden NRPS-4a, der sich aus den ersten zwei XUs der SzeS und den letzten drei XUs der GxpS zusammensetzte, wurde in den aufgenommenen HPLC-MS Daten nach m/z-Werten von 634,38 [M+H⁺] für das Phenylalanin-Derivat **10** (Formyl-lTflL) und 601,36 [M+H⁺] für das Leucin-Derivat **11** (Formyl-lTllL) gesucht. Als Vergleichssysteme diente das Konstrukt ohne N-terminalen SZ (NRPS-4b) sowie das kovalent verknüpfte System (NRPS-4c). Sowohl die Masse von **10** als auch die Masse von **11** konnten im *extracted-ion chromatogram* (EIC) der Messdaten detektiert werden, die jeweils bei einer Retentionszeit von 8 min und 7,8 min eluierten. Dabei wurde Peptid **10** am häufigsten und Peptid **11** in relativen Werten von 8% ermittelt (Abb. 3). Weiter ergaben die Messdaten der Negativkontrolle (NRPS-4b) eine signifikante Abnahme von **10** und **11** um ∼80%. Auch das kovalent verknüpfte Konstrukt (NRPS-4c) produzierte die Peptide in signifikant geringeren Mengen. So wurde **10** nur noch zu ∼60% und **11** nur noch zu ∼30% relativ zu NRPS-4a detektiert.

Letztlich zeigten die HPLC-MS Daten des dritten Hybriden (NRPS-5a), der sich aus Teilen der XldS und GxpS zusammensetzte, die Produktion der meisten erwartenden Derivate (Abb. 4). Da die C1-Domäne der XldS den Einbau einer C13-, C14- oder C15-FS an das N-terminale Ende des Peptids ermöglicht, ergeben sich mit der Promiskuität der dritten GxpS A-Domäne sechs mögliche Derivate mit m/z-Werten von 830,54 [M+H⁺] **12** (13:0-qNflL), 844,55 [M+H⁺] **13** (14:0-qNflL), 858,12 [M+H⁺] **14** (15:0-qNflL), 796,55 [M+H⁺] (13:0-qNllL), 810,57 [M+H⁺] **15** (14:0-qNllL) und 824,59 [M+H⁺] (15:0-qNllL). Vier von ihnen wurden detektiert. Die Retentionszeiten der C13-, C14- oder C15-Derivate beliefen sich auf jeweils 11,3 min, 11,8 min und 12,3 min. Während **13** das am häufigsten produzierte Peptid war, wurden die restlichen Peptide in relativen Mengen zwischen 2,3% (**12**) und 14,3% (**15**) detektiert (Abb. 9). Weiter war die Signalintensität der EICs gering, was auf eine insgesamt niedrigen Produktion hindeutet. Außerdem zeigte die Negativkontrolle (NRPS-5b) keinen signifikanten Unterschied in der Peptidproduktion zum NRPS-5a Konstrukt (Abb. 4).

### Beispiel 3: Strategien für die SYNZIP vermittelte Rekonstruktion von NRPSs

Oben wurde auf Grundlagen des XU-Konzepts das konservierte WNATE-Motiv (SEQ ID Nr: 3) des C-A Linkers als bevorzugte Schnittstelle gewählt. Diese Schnittstelle wurde von Bozhüyük et al. aus Sequenzalignments von NRPS-Linkerregionen aus *Photorhabdus* und *Xenorhabdus* sowie aus veröffentlichte NRPS-Strukturdaten anderer Organismen als idealer Fusionspunkt postuliert. Weiter wurde genannt, dass die A-T und T-C Linkerregionen aufgrund ihrer, im Vergleich zum C-A Linker, geringen konservierten Sequenz weniger für die Reprogrammierung von NRPS geeignet sind. Nichtsdestotrotz, sollte unter Einführung des SZ-Paars 17/18 in die T-C und T-A Linkerregionen vergleichend geprüft werden, ob diese Insertionsstellen nicht ebenso geeignete Fusionspunkte darstellen. Die Überprüfung dieser Hypothese erfolgte mittels des XtpS-Modellsystems. XtpS wurde dafür mit den 2017 veröffentlichten Struktur-Daten der Bacillibactin-Synthetase aus Bacillus subtilis (Tarry et al., 2017) alignt, um Rückschlüsse über mögliche Sekundärstrukturen zu erhalten. Anschließend wurden anhand dessen Schnittstellen im T-C und A-T Linkerbereich definiert, die sich letztlich auf die Sequenzmotive RV|LP (SEQ ID Nr: 4) des T-C Linkers und VY|AAP (SEQ ID Nr: 5; senkrechter Stricht verdeutlicht Schnittstelle) des A-T Linkers beliefen.

Des Weiteren sollten zwei verschiedene SYNZIP-Oligomerisierungsstatus verglichen werden, die bedingen, dass die an die SZs gebundenen XtpS-Untereinheiten entweder in räumlicher Nähe oder weiter auseinander liegen. Grundsätzlich lassen sich beide Orientierungen sowohl mit parallelen als auch mit antiparallelen SZ umsetzen. Praktikabel ist jedoch nur die Konformation, bei der die Proteine weiter auseinander liegen. Der Grund hierfür ist, dass nach Zweiteilung des NRPS-Systems die SZs nur an zwei (C-Terminus des ersten und N-Terminus des zweiten NRPS-Teils), statt an vier möglichen Termini (N- und C-Terminus des ersten sowie N- und C-Terminus des zweiten Proteinteils) der Proteine gehängt werden können. Durch die Einführung von SZ19 und der funktionalen reversen Form des SZ18 scheint jedoch eine nahe Orientierung möglich. Nichtsdestotrotz ist dieses Paar nicht durch Thompson et al. charakterisiert (lediglich SZ19 mit der forward-Form von SZ18) und dementsprechend fehlen Daten über den Kd -Wert, Interaktionspartnern etc.. Letztlich wurde das antiparallele SZ-Paar 17/18 für die weite und das parallel SZ-Paar 19/18 für die nahe Konformation genutzt.

Für die im T-C und A-T Linker geteilten XtpS (NRPS-13 und NRPS-14, Schnittstelle s. 3.2) wurden jeweils zwei Plasmide, pNA2 (xtpS_A1-T2_SZ17) und pNA3 (SZ18_xtpS_C3-TE) sowie pNA4 (xtpS_A1-A2_SZ17) und pNAs (SZ18_xtpS_T2-TE), assembliert und gemeinsam in E. coli DH10B::mtaA transformiert. Für die Repräsentation der Schnittstelle im C-A Linker wurden dagegen die Plasmide pJW61 (xtpS_A1-C3_SZ17) und pJW62 (SZ18_xtpS-A3-TE) verwendet. Weiterhin wurde für die im C-A Linker geteilte NRPS-16 der C-terminale SZ17 gegen SZ19 ersetzt, während der N-terminale SZ18reverse unverändert blieb. Als Referenz diente die Produktion durch die Wildtyp-XtpS (NRPS-1). Produktionskulturen aller Konstrukte wurden gleichzeitig als Triplikate angesetzt und die Synthese von 1 und 2 wurde mittels HPLC-MS geprüft.

Da aufgrund eines fehlenden Standards eine absolute Quantifizierung nicht möglich war, erfolgte eine relative Auswertung der Peak Area (Abb. 5). Anders als aus den relativen Werten in Abb. 5 zu erwarten wäre, wird das lineare Peptid 1 bezogen auf die absolute Peptidausbeute nicht in nahezu gleichen Mengen wie das zyklische Peptid 2 gebildet, sondern nur zu etwa 0,1%. Dieses Ergebnis kommt lediglich aufgrund der besseren Ionisierung des linearen Peptids zustande und muss bei Betrachtung der relativen Werte berücksichtig werden. Die Messergebnisse zeigten, dass für alle Konstrukte die Produktion des zyklischen Produkts 2, mit einem m/z-Wert von 411,31 [M+H+], und in den meisten Fällen auch die Produktion des linearen Peptids 1, mit einem m/z-Wert von 429,31 [M+H+], nachgewiesen werden konnte (Abb. 5). 2 wurde mit etwa 80% relativ zum WT am besten durch die im T-C (NRPS-13) und A-T (NRPS-14) Linker geteilten Konstrukte produziert, wohingegen die beiden im C-A geteilten Konstrukte, NRPS-15 und NRPS-16, mit 27% (NRPS-15) und 13% (NRPS-16) eine deutlich schlechtere Produktion zeigten. Das lineare Peptid (1) wurde in vernachlässigbar geringeren Mengen besser durch NRPS-14 statt NRPS-13 produziert und NRPS-16 zeigte gar keine Produktion von 1.

### Beispiel 5: Einfluss der SYNZIPs auf die Produktion der A-T geteilten XtpS

Der Einfluss der SZs auf die Produktion von 1 und 2 wurde für das A-T geteilte Konstrukt NRPS-14a (Abb. 6) geprüft. Nach Assemblierung von pNA11 (xtpS_A1-A2) und pNA12 (xtpS_T2-TE) wurden Kontrollexperimente durchgeführt, bei welchen im ersten Fall der N-terminale (NRPS-14b), im zweiten Fall der C-terminale (NRPS-14c) und im dritten Fall beide SZs (NRPS-14c) (Abb. 6) weggelassen wurden. Hierfür wurden die Plasmide pNA12 (xtpS_T2-TE) und pNA11 (XtpS_A1-A2) kloniert, denen jeweils die Sequenz des SZ17 und SZ18 fehlte. Die Ergebnisse der HPLC-MS Daten sind in Abb. 6 gezeigt. Die Negativkontrollen des A-T geteilten Konstrukts (NRPS-14b, NRPS-14c und NRPS-14d) zeigten mit ∼3-10% des WT-Levels eine sehr geringe Produktion des zyklischen Produkts 2 und absolut keine Produktion des linearen Peptids 1. Insgesamt zeigten die Kontrollen eine Abnahme der Produktivität von ≥ 90% im Verhältnis zu NRPS-14a (Abb. 6). Des Weiteren produzierte NRPS-14a die Peptide 2 und 1 mit 104% und 81% auf WT-Niveau.

### Beispiel 6: Einfluss der SYNZIPs auf die Produktion der A-C (SZ19/18) geteilten XtpS

Die gleichen Kontrollexperimente wurden ebenfalls für das A-C geteilte Konstrukt mit SZ-Paar 19/18 durchgeführt. Da dieses Konstrukt bereits mit beiden SZs eine niedrige Produktion zeigte (Abb. 5), konnte für die drei durchgeführten Kontrollexperimente ebenfalls nur eine sehr geringe oder keine Produktion von 2 und 1 detektiert werden (Abb. 7). Die relative Auswertung der HPLC-MS Messdaten zeigte keine Peptidproduktion für Kontrollexperimente NRPS-16b und NRPS-16d, NRPS-16c zeigte mit 3,2% des WT-Level nur eine sehr geringe Produktion von 2. Im Verhältnis zum Konstrukt mit beiden SZs (NRPS-16a), weist Kontrolle NRPS-16c eine Abnahme der Produktion des zyklischen Peptids 2 um 80% auf.

### Beispiel 7: Einfluss von GS-Linkern auf die Produktion der A-C (SZ17/18) geteilten XtpS

Da das C-A (NRPS-15, Abb. 5), im Vergleich zum T-C (NRPS-13, Abb. 5) und A-T (NRPS-14, Abb. 5) geteilten XtpS-Konstrukt, eine deutlich schlechtere Produktivität zeigte, wurde zwischen dem C-terminale Ende des ersten XtpS-Abschnitts und SZ17, GS-Linker unterschiedlicher Länge eingeführt, mit dem Ziel die Produktivität zu steigern. Da nach dem von Bozhüyük et al. veröffentlichten XU-Konzepts zur Konstruktion von reprogrammierter NRPS, zehn AS des konservierten WNATE-Motivs deletiert werden und ebenso für das in Abb. 8 gezeigte C-A Konstrukt (NRPS-15) verfahren wurde, wurde mit der Einführung eines zehn AS langen GS-Linkers begonnen. Dies wurde durch die Assemblierung des Plasmids pNA8 (xtpS_A1-C3_GS(10)_SZ17), einem von pJW61 (A1-C3_SZ17) abgeleitetem Plasmid, realisiert. Zusätzlich wurden zwei weitere Plasmide, pNA9 (xtpS_A1-C3_GS(8)_SZ17) und pNA10 (xtpS_A1-C3_GS(4)_SZ17) konstruiert, die für einen acht und vier AS lange GS-Linker codieren. Die Auswertung der HPLC-MS Messdaten zeigten eine bessere Produktion aller Konstrukte (NRPS-15b, NRPS-15c, NRPS-15d) mit GS-Linker gegenüber jenem Konstrukt ohne. Insgesamt führte die Einführung eines Linkers zu einer durchschnittliche Erhöhung der Produktivität um ∼ 37% für das zyklische 2 und ∼26% für das lineare Peptid 1. Des Weiteren wurde das zyklische Produkt 2 mit nahezu WT-Niveau für alle Konstrukte mit GS-Linker produziert.

### Beispiel 7: Konzeptnachweis: Produktivität eines dreigeteilten XtpS-Systems

Da die Zweiteilung der XtpS für jede der bevorzugt genannte Position erfolgreich war, galt es im nächsten Schritt das System an zwei Positionen zu teilen. Hierfür wurde ein weiteres SYNZIP Paar, SZ1 und SZ2, eingeführt, das mit SZ17 und SZ18 nicht kommuniziert und dadurch ein sogenanntes orthogonales Netzwerk bildet. Mit einem Kd-Wert von < 10 nM zeigen SZ1 und SZ2, wie auch SZ17 und SZ18, eine sehr starke Affinität zueinander. Weiter wurden als Positionen für die Dreiteilung ausschließlich die A-T und T-C Linkerregionen gewählt, die sich durch NRPS-13 und NRPS-14 als günstigste Positionen mit der besten Produktion herausstellten (Abb. 5). Dementsprechend wurden zwei Konstrukte, NRPS-17a und NRPS-18a, erzeugt, die jeweils unter Einführung der zwei SZ-Paare in den Linker-Bereichen T-C bzw. A-T geteilt wurden. Im Detail wurden die SZs in den zweiten und dritten T-C Linker bzw. zweiten und dritten A-T Linker eingebracht. Insgesamt wurden hierfür vier weitere Plasmide, namentlich pNA17 (SZ18_xtpS_C3-T3_SZ1) und pNA18 (SZ2_xtpS_C4-TE), für die Teilung im T-C, sowie pNA15 (SZ18_xtpS_T2-A3_SZ1) und pNA16 (xtpS_SZ2_T3-TE), für die Teilung im A-T Bereich, kloniert. Außerdem wurden als Negativkontrollen die Plasmide ohne SZs assembliert. Hieraus ergaben sich pNA19 (xtpS_T2-A3) und pNA20 (xtpS_T3-TE) für die Negativkontrolle des A-T Splits (NRPS-18b, Abb. 9).

Für beide dreigeteilten Konstrukte, NRPS-17a und NRPS-18a, konnte die Produktion des linearen 1 und zyklischen 2 Peptids identifiziert werden (Abb. 9). Dabei produzierte NRPS-17a im Vergleich zu NRPS-18a, die Peptide in zwei (2) bis mehr als dreifacher Menge (1). Dementsprechend wurde 2 zu jeweils 71,7% und 32,2% und 1 zu 25,6% und 7,3% identifiziert. Des Weiteren zeigte die Negativkontrolle des A-T geteilten Systems (NRPS-18b) keine Produktion der Peptide.

### Beispiel 8: SYNZIP vermittelter Tridomänen-Tausch für die Konstruktion von Hybrid-NRPS

Neben XtpS wurden auch GxpS (NRPS-20) dreigeteilt (Abb. 10) und es wurden Tridomänenabschnitte von XldS und SzeS erzeugt. Die dabei erhaltenen Tridomänenabschnitte der Systeme sollten dann in einem weiteren Versuch, für die Produktion neuartiger Peptide, untereinander kombiniert werden. Aus Sequenzaligments aller A-T und T-C Linkerregion der vier Systemen, stellte sich die Schnittstelle des A-T Linker (s. 3.2, leichte Variation des Sequenzmotivs innerhalb und zwischen den Systemen) als günstigere Schnittstelle heraus (Sequenzmotiv der Schnittstelle konservierter). Demnach wurde für alle weiteren gezeigten Konstrukte ausschließlich die Schnittstelle im A-T Linker genutzt. Dies bedingt, dass nicht mehr, wie im XU-Konzept, die Substratspezifität der downstream sondern die der upstream liegenden C-Domäne berücksichtigt werden sollte. NRPS-23 besteht neben XtpS noch aus Teilen der GxpS und SzeS (Abb. 11). Nach Überprüfung der Produktivität der dreigeteilten NRPS-18, NRPS-23b und NRPS-23c wurde die Tridomänen untereinander getauscht.

### Beispiel 9: Produktivität weiterer dreigeteilter Systeme

Für die Konstruktion der dreigeteilten GxpS (NRPS-18) und SzeS (NRPS- 19) Systeme, wurde jeweils ein Set aus drei Plasmiden kloniert. Hieraus ergaben sich die Plasmide pNA26 (gxpS_A1-A2_SZ17), pNA27 (SZ18_gxpS_T2-A3_SZ1) und pNA28 (SZ2_gxpS_T3-TE) sowie pNA29 (szeS_C1-A2_SZ17), pNA30 (szeS_T2-A3_SZ1) und pNA31 (SZ2_szeS_T3-TE), die jeweils gemeinsam in E. coli DH10B::mtaA transformiert wurden.

Für die NRPS-20 (dreigeteilte GxpS) konnten alle vier Derivate 3, 4, 5 und 6 mit m/z-Werten von jeweils 586,40 [M+H+], 600,41 [M+H+], 552,41[M+H+] und 566,43 [M+H+] ermittelt werden (Abb. 10). Im Vergleich zur WT-NRPS (NRPS-2) war die Produktivität jedoch stark reduziert. So wurde 3 nur noch zu 5,2%, 4 nur noch zu 10,8% und 5 und 6 nur noch zu ∼14% produziert (Abb. 10).

### Beispiel 10: Tridomänen-Tausch für die Produktion neuartiger Peptide

Die auf drei Plasmiden beruhende Teilung der beschriebenen NRPSs macht eine Manipulation der Systeme einfach. In der experimentellen Umsetzung werden anstelle der ursprünglichen Plasmide ein oder zwei ersetzt und in neuer Konstellation gemeinsam in den jeweiligen Expressionsstamm transformiert. Die posttranslationale Kommunikation zwischen den verschiedenen NRPSs wird dann durch die künstlichen Leucin-Zipper vermittelt. So kann bspw. das zweite Plasmid des XtpS-, gegen das zweite des GxpS-Sets ersetzt und dadurch ein neues Hybrid-System konstruieren werden. Insgesamt erlauben die in dieser Arbeit erzeugten Plasmide die Konstruktion von 50 Hybrid-Synthetasen. In den nachfolgenden Beispielen werden 8 von ihnen beschrieben.

Der erste Tridomänen-Tausch sollte die Substitution des zweiten Valins des Xtp gegen ein Phenylalanin ermöglichen. In der experimentellen Umsetzung wurden hierfür anstelle des pNA15 Plasmids jeweils die Plasmide pNA27 und PNA30 gemeinsam mit pNA4 und pNA16 in E. coli DH10B::mtaA transformiert und dadurch die Erzeugung der Hybriden NRPS-23b und NRPS-23c ermöglicht (Abb. 11).

Die relative Auswertung der HPLC-MS Analyse zeigte für NRPS-23b und NRPS-23c die zu erwartenden Peptide (Abb. 11). So wurde durch NRPS-23b sowohl das Phenylalanin-Derivat als auch das Leucin-Derivat in linearer (16, 17) und zyklischer Form (18, 19) detektiert. Im EIC der Peptide 16 und 19 traten des Weiteren jeweils Doppelpeaks auf, die unterschiedliche Retentionszeiten aufwiesen, jedoch eine identische Fragmentierung des MS2-Spektrums zeigten. Hieraus wurde geschlossen, dass die Peptide als Stereoisomere vorkamen und entsprechend zu unterschiedlichen Zeiten eluierten. Da ausschließlich für die letzte C/E-Domäne des Hybriden NRPS-23b ein nicht-natürliches Protein-Protein Interface besteht, wurde deduziert, dass die upstream AS in zwei Konformationen vorkommt. Im Beispiel von 16 und 19 sind dies jeweils die AS Phenylalanin und Leucin. Welche AS jedoch tatsächlich betroffen ist, wurde nicht überprüft und bleibt daher ungeklärt. Weiterhin wurden durch NRPS-23c, wie bereits durch NRPS-23b, die Peptide 16 und 18 produziert. Dabei trat für Peptid 16 erneut ein Doppelpeak auf, weswegen die Stereoisomere 16a und 16b vermutet wurden. Für die relative Auswertung der Isomere wurden die Peakflächen addiert.

Die am häufigsten detektierten Peptide stellten die linearen Peptide 16a/b (in beiden Hybriden) und 17 dar, die alle in nahezu identischen Mengen von 94,4%-100% produziert wurden (Abb. 11). Welchen Einfluss die Ionisierung auf die Häufigkeit des linearen Peptids hat wird in Abschnitt 4 diskutiert. Insgesamt zeigten beide Hybriden, NRPS-23b und NRPS-23c eine ähnliche Produktion der Peptide 16a/b und 18, wonach 16a/b mit jeweils 94,4% und 100% und 18 mit jeweils 19,1% und 24,8% produziert wurden. Des Weiteren konnten für die Phenylalanin- (16a/b und 18) und Leucin-Derivate (17a und 19a/b), die durch NRPS-23b produziert wurden, ebenfalls sehr ähnliche Werte ermittelt werden.

Beim zweiten Tridomänen-Tausch sollte die Substitution des Phenylalanins des Gxps durch das Valin (aus Xtp) oder Phenylalanin (aus Sze) erfolgen. Indem pNA27 gegen jeweils pNA15 und pNA30 ersetzt wurde, wurden die Hybriden NRPS-24a und NRPS-24c erzeugt (Abb. 12).

Die relative Auswertung der Messdaten ergab, dass für NRPS-22a und NRPS-22c Peptidproduktion ermittelt werden konnte. Hierbei produzierten beide Hybride sowohl das Valin- (20, 22, 24 und 3) als auch das Leucin-Derivat (21a/b, 23, 25 und 4) in linearer und zyklischer Form (Abb. 12). Das am häufigsten produzierte Peptid des Hybrid NRPS-24a war das zyklische Valin-Derivat 22. Die lineare Form (20) wurde mit 34,7% relativ zu 22 detektiert. Weiter wurde das Leucin-Derivat in zyklischer Form (23) mit 62,9% in geringeren Mengen als 22 produziert. Das lineare Peptid 21 kam mit relativen Werten von 20,9% vor und wurde als Stereoisomer (21a und 21b) detektiert. NRPS-24c zeigte im Vergleich zu NRPS-24a eine insgesamt schlechtere Produktion. So wurde das zyklische Valin-Derivat 3 im Vergleich zu 22 zu 66,1% produziert und das zyklische Leucin-Derivat 4 wurde in relativen Werten nur noch zu 16,3% detektiert. Des Weiteren wurde in NRPS-22C mit 13,4% und 2,7% die linearen Peptide 24 und 25 in geringeren Mengen detektiert als die zyklischen Peptide. Die Strukturen der Peptide sind in Abb. 12B gezeigt. Weitere neue Peptide konnten entsprechend durch weitere Kombinationen der entsprechenden Plasmide erhalten werden und sind in den Abbildungen 14 und 14 gezeigt.

Weiterhin wurde in Abbildung 15 gezeigtes Hybrid von PKS und NRPS Modulen generiert, die zu einer vollständigen Synthese des Glidobactin Peptids führt. Dazu wurde GlbD zwischen A-T mit SZ17/SZ18 geteilt. In den Negativkontrollen ohne jeweils einen oder beide SZs, gibt es praktisch keine Glidobactin A Produktion.

Folgende Plasmide wurden in Kontext der Beispiele verwendet:

| **Name** | **Genotyp** | **Referenz** |
|---|---|---|
| pACYC_ara_araE | ori P15A, cm^{R}, *araC-P_{BAD}*, *tac*I-*araE,* MCS | AK Bode |
| pCOLA_ara_tacI | ori ColA, kan^{R}, *araC-P_{BAD}*, *tac*I, MCS | AK Bode |
| pCDF_ara_tacI | ori ColDF13, spek^{R}, *araC-P_{BAD} tac*I, MCS | AK Bode |
| pACYC_ara_XtpS | ori P15A, cm^{R}, *araC-P_{BAD} XtpS* und *tac*I*-araE* | Watzel, 2019 (nicht veröffentlicht) |
| pACYC_ara_GxpS | ori P15A, cm^{R}, *araC-P_{BAD} GxpS* und *tac*I*-araE* | Watzel, 2019 (nicht veröffentlicht) |
| pA22.3 | ori ColA, kan^{R}, *araC-P_{BAD} szeS_* C₁A₁T₁C/E₂A₂T₂C₃ _*gxpS_* A₃T₃C/E₄A₄T₄TE und *tac*I | Bozhüyük et al., 2018 |
| pJW61 | ori P15A, cm^{R}, *araC-P_{BAD} xtpS_* A₁T₁C/E₂A₂T₂C₃-SYNZIP17 und *tac*I*-araE* | Watzel, 2019 (nicht veröffentlicht) |
| pJW62 | ori ColA, kan^{R}, *araC-P_{BAD}* SYNZIP18-*xtpS_* A₃T₃C/E₄A₄T₄TE und tacl | Watzel, 2019 (nicht veröffentlicht) |
| pJW63 | ori P15A, cm^{R}, *araC-P_{BAD} xtpS_* A₁T₁C/E₂A₂T₂C₃ und *tac*I*-araE* | Watzel, 2019 (nicht veröffentlicht) |
| pJW64 | ori ColA, kan^{R}, *araC-P_{BAD} xtpS_* A₃T₃C₇E₄A₄T₄TE und *tac*I | Watzel, 2019 (nicht veröffentlicht) |
| PJW75 | ori P15A, cm^{R}, *araC-P_{BAD} gxpS_* A₁T₁C/E₂A₂T₂C₃-SYNZIP17 und *tac*I*-araE* | Watzel, 2019 (nicht veröffentlicht) |
| pJW76 | ori ColA, kan^{R}, araC-P_{BAD} SYNZIP18-*gxpS_* A₃T₃C/E₄A₄T₄TE und tacl | Watzel, 2019 (nicht veröffentlicht) |
| pJW82 | ori P15A, cm^{R}, *araC-P_{BAD} gxpS_* A₁T₁C/E₂A₂T₂C₃ und *tac*I*-araE* | Watzel, 2019 (nicht veröffentlicht) |
| pJW83 | ori ColA, kan^{R}, *araC-P_{BAD} gxpS_* A₃T₃C/E₄A₄T₄TE und *tac*I | Watzel, 2019 (nicht veröffentlicht) |
| pJW91 | ori P15A, cm^{R}, *araC-P_{BAD} ambS_* A₁T₁C/E₂A₂T₂C₃-SYNZIP17 und *tac*I*-araE* | Diese Arbeit |
| pJW92 | ori P15A, cm^{R}, *araC-P_{BAD} szeS_*C₁A₁T₁C₁E₂A₂T₂C₃-SYNZIP17 und *tac*I*-araE* | Diese Arbeit |
| pJW93 | ori P15A, cm^{R}, *araC-P_{BAD} xldS_*C₁A₁T₁C/E₂A₂T₂C₃-SYNZIP17 und *tac*I*-araE* | Diese Arbeit |
| pNA1 | ori P15A, cm^{R}, *araC-P_{BAD} xtpS_* A₁T₁C/E₂A₂T₂C₃-SYNZIP19 und *tac*I*-araE* | Diese Arbeit |
| pNA2 | ori p15A, cm^{R}, *araC-P_{BAD} xtpS_* A₁T₁C/E₂A₂T₂-SYNZIP17 und *tac*I*-araE* | Diese Arbeit |
| pNA3 | ori ColA, kan^{R}, *araC-P_{BAD}* SYNZIP18-*xtpS_* C₃A₃T₃C/E₄A₄T₄TE und tacl | Diese Arbeit |
| pNA4 | ori p15A, cm^{R}, *araC-P_{BAD} xtpS_* A₁T₁C/E₂A₂-SYNZIP₁₇ und *tac*I*-araE* | Diese Arbeit |
| pNA5 | ori ColA, kan^{R}, *araC-P_{BAD}* SYNZIP18-*xtpS*_ T₂C₃A₃T₃C/E₄A₄T₄TE und tacl | Diese Arbeit |
| pNA6 | ori p15A, cm^{R}, *araC-P_{BAD} xtpS_* A₁T₁C/E₂A₂T₂ und *tac*I-*araE* | Diese Arbeit |
| pNA7 | ori ColA, kan^{R}, *araC-P_{BAD} xtpS_* C₃A₃T₃C/E₄A₄T₄TE und *tac*I | Diese Arbeit |
| pNA8 | ori p15A, cm^{R}, *araC-P_{BAD} xtpS_* A₁T₁C/E₂A₂T₂C₃-GS(10)-SYNZIP17 und *tac*I*-araE* | Diese Arbeit |
| pNA9 | ori p15A, cm^{R}, *araC-P_{BAD} xtpS_* A₁T₁C/E₂A₂T₂C₃-GS(8)-SYNZIP17 und *tac*I*-araE* | Diese Arbeit |
| pNA10 | ori p15A, cm^{R}, *araC-P_{BAD} xtpS_* A₁T₁C/E₂A₂T₂C₃-GS(4)-SYNZIP17 und *tac*I*-araE* | Diese Arbeit |
| pNA11 | ori p15A, cm^{R}, *araC-P_{BAD} xtpS_* A₁T₁C/E₂A₂ und tacl*araE* | Diese Arbeit |
| pNA12 | ori ColA, kan^{R}, *araC-P_{BAD} xtpS_* T₁C₃A₃T₃C/E₄A₄T₄TE und *tac*I | Diese Arbeit |
| pNA14 | ori p15A, cm^{R}, *araC-P_{BAD} xtpS_* A₁T₁C/E₂A₂T₁C₃A₃T₃C/E₄A₄T₄-*gxpS*_TE und *tac*I*-araE* | Diese Arbeit |
| pNA15 | ori ColA, kan^{R}, *araC-P_{BAD}* SYNZIP18-*xtpS*_T₂C₃A₃-SYNZIP1 und tacl | Diese Arbeit |
| pNA16 | ori CloDF13, spec^{R}, *araC-P_{BAD}* SYNZIP2-*xtpS*_T_{H}C/E₄A₄T₄TE und *tac*I | Diese Arbeit |
| pNA17 | ori ColA, kan^{R}, *araC-P_{BAD}* SYNZIP18-*xtpS*_C₃A₃T₄-SYNZIP1 und tacl | Diese Arbeit |
| pNA18 | ori CloDF13, spec^{R}, *araC-P_{BAD}* SYNZIP2-*xtpS*_C/E₄A₄T₄TE und *tac*I | Diese Arbeit |
| pNA19 | ori ColA, kan^{R}, *araC-P_{BAD} xtpS*_T₂C₃A₃ und tacl | Diese Arbeit |
| pNA20 | ori CloDF13, spec^{R}, *araC-P_{BAD} xtpS_* T₃C/E₄A₄T₄TE und *tac*I | Diese Arbeit |
| pNA21 | ori ColA, kan^{R}, *araC-P_{BAD} xtpS_*C₃A₃T₄ und *tac*I | Diese Arbeit |
| pNA22 | ori CloDF13, spec^{R}, *araC-P_{BAD} xtpS_*C/E₄A₄T₄TE und *tac*I | Diese Arbeit |
| pNA26 | ori p15A, cm^{R}, *araC-P_{BAD} gxpS*_A₁T₁C/E₂A₂-SYNZIP17 und *tac*I*-araE* | Diese Arbeit |
| pNA27 | ori ColA, kan^{R}, *araC-P_{BAD}* SYNZIP18-*gxpS*_T₂C₃A₃-SYNZIP1 und *tac*I | Diese Arbeit |
| pNA28 | ori CloDF13, spec^{R}, *araC-P_{BAD}* SYNZIP2-*gxpS*_T₃C/E₄A₄T₄ C/E₅A₅T₅TE und *tac*I | Diese Arbeit |
| pNA29 | ori p15A, cm^{R}, *araC-P_{BAD} sze*S_ C₁A₁T₁C/E₂A₂-SYNZIP17 und *tac*I*-araE* | Diese Arbeit |
| PNA30 | ori ColA, kan^{R}, *araC-P_{BAD}* SYNZIP18-*szeS*_T₂C₃A₃-SYNZIP1 und *tac*I | Diese Arbeit |
| PNA31 | ori CloDF13, spec^{R}, *araC-P_{BAD}* SYNZIP2-*szeS*_T₃C/E₄A₄T₄ C/E₅A₅T₅C₆A₆T₆TE und *tac*I | Diese Arbeit |
| pNA34 | ori ColA, kan^{R}, *araC-P_{BAD}* SYNZIP18-*xtpS_*A₃T₃C/E₄A₄T₄-*gxpS*_TE und *tac*I | Diese Arbeit |

## Patentansprüche

1. **Ein Protein oder ein Proteinfragment,** umfassend mindestens eine erste Domäne oder Teildomäne einer Nicht-Ribosomalen-Peptidsynthetase (NRPS), einer Polyketidsynthase (PKS) oder einer NRPS/PKS-Hybridsynth(et)ase (erste PKS-NRPS-Domäne), wobei das Protein oder das Proteinfragment einen N-Terminus oder einen C-Terminus umfassend eine erste Bindedomäne aufweist und wobei diese erste Bindedomäne vorzugsweise den N-Terminus, respektive C-Terminus, des Proteins oder des Proteinfragments darstellt, und wobei die erste Bindedomäne durch die Eigenschaft gekennzeichnet ist eine spezifische Protein-Protein Bindung mit mindestens einer korrespondierenden zweiten Bindedomäne eingehen zu können.

2. Das Protein oder Proteinfragment nach Anspruch 1, weiter umfassend mindestens eine, vorzugsweise zwei, drei oder vier oder mehr, weitere PKS-NRPS-Domäne(n), wobei die weitere(n) PKS-NRPS-Domäne(n) in direkter funktioneller Anordnung neben der ersten PKS-NRPS-Domäne angeordnet ist (sind).

3. Das Protein oder Proteinfragment nach einem der Ansprüche 1 oder 2, wobei die erste PKS-NRPS-Domäne, oder Teildomäne, ausgesucht wird aus einer A-Domäne, einer C-Domäne, einer C/E-Domäne, einer E Domäne, einer Cₛₜₐᵣₜ-Domäne, einer FT-Domäne, oder einer T-Domäne.

4. Das Protein oder Proteinfragment nach einem der Ansprüche 1 bis 3, umfassend mindestens eine A-Domäne, eine C-Domäne und eine T-Domäne, vorzugsweise wobei das Protein oder Proteinfragment mindestens ein NRPS-PKS Elongationsmodul, ein Initiierungsmodul, oder ein Terminierungsmodul aufweist.

5. Das Protein oder Proteinfragment nach einem der Ansprüche 1 bis 4, wobei die Bindedomäne eine synthetische coiled-coil Domäne (SYNZIP) umfasst, vorzugsweise wobei der SYNZIP aus einem SYNZIP 1-23 ausgewählt ist.

6. Das Protein oder Proteinfragment nach einem der vorstehenden Ansprüche, wobei der der ersten Bindedomäne entgegengesetzte Terminus eine dritte Bindedomäne umfasst, und wobei die dritte Bindedomäne durch die Eigenschaft gekennzeichnet ist eine spezifische Protein-Protein Bindung mit mindestens einer korrespondierenden vierten Bindedomäne eingehen zu können.

7. Das Protein oder Proteinfragment nach Anspruch 6, wobei die erste und zweite Bindedomäne gezielt eine Bindung mit der dritten oder vierten Bindedomäne eingehen können.

8. Das Protein oder Proteinfragment nach einem der Ansprüche 1 bis 7, wobei die erste Bindedomäne durch einen Linker mit der ersten PKS-NRPS-Domäne verbunden ist.

9. **Ein isoliertes Nukleinsäurekonstrukt,** umfassend einen ersten kodierenden Bereich, der eine Nukleinsäuresequenz aufweist, die für ein Protein oder Proteinfragment nach einem der Ansprüche 1 bis 8 kodiert.

10. **Ein Vektorsystem zur Herstellung einer funktionellen NRPS oder PKS,** wobei das Vektorsytem mindestens ein Nukleinsäurekonstrukt gemäß Anspruch 9 umfasst, und wobei das mindestens ein Nukleinsäurekonstrukt geeignet ist mindestens zwei Proteine oder Proteinfragmente gemäß einem der Ansprüche 1 bis 8 zu exprimieren, und wobei die mindestens zwei Proteine oder Proteinfragmente unterschiedlich sind und zusammen eine funktionelle NRPS, PKS oder NRPS/PKS-Hybrid bilden.

11. Das Vektorsystem nach Anspruch 10, wobei die mindestens zwei Proteine oder Proteinfragmente über die Bindung der ersten und zweiten Bindedomäne die funktionelle NRPS, PKS oder NRPS/PKS-Hybrid bilden.

12. Das Vektorsystem nach einem der Ansprüche 10 oder 11, wobei das Vektorsystem Nukleinsäurekonstrukte umfasst, die geeignet sind für die Expression von mindestens drei oder mehr Proteinen oder Proteinfragmenten gemäß eines der Ansprüche 1 bis 8, oder wobei mindestens zwei der drei oder mehr Proteinen oder Proteinfragmenten zusammen eine funktionelle NRPS, PKS oder ein NRPS/PKS-Hybrid bilden.

13. Das Vektorsystem nach Anspruch 12, wobei die mindestens drei Proteine oder Proteinfragmente gemeinsam eine funktionelle NRPS, PKS oder NRPS/PKS-Hybrid bilden, und wobei die funktionelle NRPS oder PKS über Bindung der Proteine oder Proteinfragmente untereinander mittels Bindung einer ersten zu einer zweiten Bindedomäne und Bindung einer dritten zu einer vierten Bindedomäne gebildet wird.

14. **Eine Verfahren zur Herstellung einer funktionellen (vollständigen) NRPS oder PKS,** umfassend das in-Kontakt bringen von mindestens einem ersten Protein oder Proteinfragment gemäß einem der Ansprüche 1 bis 8 mit einem zweiten Protein oder Proteinfragment gemäß einem der Ansprüche 1 bis 8, wobei das erste Protein oder Proteinfragment eine terminale erste Bindedomäne aufweist, und wobei das zweite Protein oder Proteinfragment anstatt der terminalen ersten Bindedomäne die terminale zweite Bindedomäne aufweist.


SEQUENCE LISTING

<110>  Johann Wolfgang Goethe-Universität Frankfurt am Main

<120>  ADAPTERSYSTEM ARTIFIZIELLER NICHTRIBOSOMALER PEPTIDSYNTHETASEN
       UND POLYKETIDSYNTHASEN

<130>  U30947EP

<160>  30

<170>  PatentIn version 3.5

<210>  1
<211>  42
<212>  PRT
<213>  Artificial Sequence

<220>
<223>  SYNZIP 17

<400>  1

Asn Glu Lys Glu Glu Leu Lys Ser Lys Lys Ala Glu Leu Arg Asn Arg
1               5                   10                  15

Ile Glu Gln Leu Lys Gln Lys Arg Glu Gln Leu Lys Gln Lys Ile Ala
            20                  25                  30

Asn Leu Arg Lys Glu Ile Glu Ala Tyr Lys
        35                  40

<210>  2
<211>  42
<212>  PRT
<213>  Artificial Sequence

<220>
<223>  SYNZIP 18

<400>  2

Ser Ile Ala Ala Thr Leu Glu Asn Asp Leu Ala Arg Leu Glu Asn Ala
1               5                   10                  15

Arg Leu Glu Lys Asp Ile Ala Asn Leu Ala Lys Leu Glu Arg Glu Glu
            20                  25                  30

Ala Tyr Glu Ala Tyr Glu Ala Tyr Glu Phe
        35                  40

<210>  3
<211>  5
<212>  PRT
<213>  Artificial Sequence

<220>

<223>  NRPS Verbindungsmotiv

<400>  3

Trp Asn Ala Thr Glu
1               5


<210>  4
<211>  4
<212>  PRT
<213>  Artificial Sequence

<220>
<223>  Sequenzmotiv

<400>  4

Arg Val Leu Pro
1


<210>  5
<211>  5
<212>  PRT
<213>  Artificial Sequence

<220>
<223>  Verbindungsmotiv

<400>  5

Val Tyr Ala Ala Pro
1               5


<210>  6
<211>  5
<212>  PRT
<213>  Artificial Sequence

<220>
<223>  NRPS Peptidsequenz

<400>  6

Ser Gln Phe Leu Leu
1               5


<210>  7
<211>  5
<212>  PRT
<213>  Artificial Sequence

<220>
<223>  NRPS Peptidsequenz

<400>  7

Ser Gln Leu Leu Leu
1               5

<210>  8
<211>  5
<212>  PRT
<213>  Artificial Sequence

<220>
<223>  NRPS Peptidsequenz

<400>  8

Leu Thr Phe Leu Leu
1               5


<210>  9
<211>  5
<212>  PRT
<213>  Artificial Sequence

<220>
<223>  NRPS Peptidsequenz

<400>  9

Leu Thr Leu Leu Leu
1               5


<210>  10
<211>  5
<212>  PRT
<213>  Artificial Sequence

<220>
<223>  NRPS Peptidsequenz

<400>  10

Gln Asn Phe Leu Leu
1               5


<210>  11
<211>  5
<212>  PRT
<213>  Artificial Sequence

<220>
<223>  NRPS Peptidsequenz

<400>  11

Gln Asn Leu Leu Leu
1               5


<210>  12
<211>  4
<212>  PRT
<213>  Artificial Sequence

<220>

<223>  NRPS Peptidsequenz

<400>  12

Val Leu Val Val
1


<210>  13
<211>  5
<212>  PRT
<213>  Artificial Sequence

<220>
<223>  NRPS Peptidsequenz

<400>  13

Val Leu Phe Leu Leu
1               5


<210>  14
<211>  5
<212>  PRT
<213>  Artificial Sequence

<220>
<223>  NRPS Peptidsequenz

<400>  14

Leu Leu Phe Leu Leu
1               5


<210>  15
<211>  5
<212>  PRT
<213>  Artificial Sequence

<220>
<223>  NRPS Peptidsequenz

<400>  15

Val Leu Leu Leu Leu
1               5


<210>  16
<211>  5
<212>  PRT
<213>  Artificial Sequence

<220>
<223>  NRPS Peptidsequenz

<400>  16

Leu Leu Leu Leu Leu
1               5

<210>  17
<211>  4
<212>  PRT
<213>  Artificial Sequence

<220>
<223>  NRPS Peptidsequenz

<400>  17

Val Leu Phe Val
1


<210>  18
<211>  4
<212>  PRT
<213>  Artificial Sequence

<220>
<223>  NRPS Peptidsequenz

<400>  18

Val Leu Leu Val
1


<210>  19
<211>  5
<212>  PRT
<213>  Artificial Sequence

<220>
<223>  NRPS Peptidsequenz

<400>  19

Val Leu Val Leu Leu
1               5


<210>  20
<211>  5
<212>  PRT
<213>  Artificial Sequence

<220>
<223>  NRPS Peptidsequenz

<400>  20

Leu Leu Val Leu Leu
1               5


<210>  21
<211>  5
<212>  PRT
<213>  Artificial Sequence

<220>

<223>  NRPS Peptidsequenz

<400>  21

Val Leu Val Leu Leu
1               5

<210>  22
<211>  6
<212>  PRT
<213>  Artificial Sequence

<220>
<223>  NRPS Peptidsequenz

<400>  22

Val Leu Val Val Tyr Trp
1               5

<210>  23
<211>  4
<212>  PRT
<213>  Artificial Sequence

<220>
<223>  NRPS Peptidsequenz

<400>  23

Val Leu Phe Val
1

<210>  24
<211>  4
<212>  PRT
<213>  Artificial Sequence

<220>
<223>  NRPS Peptidsequenz

<400>  24

Leu Leu Phe Val
1

<210>  25
<211>  4
<212>  PRT
<213>  Artificial Sequence

<220>
<223>  NRPS Peptidsequenz

<400>  25

Val Leu Leu Val
1

<210>  26
<211>  4
<212>  PRT
<213>  Artificial Sequence

<220>
<223>  NRPS Peptidsequenz

<400>  26

Leu Leu Leu Val
1


<210>  27
<211>  6
<212>  PRT
<213>  Artificial Sequence

<220>
<223>  NRPS Peptidsequenz

<400>  27

Val Leu Phe Val Tyr Trp
1               5


<210>  28
<211>  6
<212>  PRT
<213>  Artificial Sequence

<220>
<223>  NRPS Peptidsequenz

<400>  28

Leu Leu Phe Val Tyr Trp
1               5


<210>  29
<211>  6
<212>  PRT
<213>  Artificial Sequence

<220>
<223>  NRPS Peptide Sequence

<400>  29

Val Leu Leu Val Tyr Trp
1               5


<210>  30
<211>  6
<212>  PRT
<213>  Artificial Sequence

<220>

<223>  NRPS Peptidsequenz

<400>  30

Leu Leu Leu Val Tyr Trp
1               5